# EUROPEAN PATENT SPECIFICATION

(11) **EP 3 010 055 B1**
(45) Date of publication and mention of the grant of the patent: **20.10.2021**
(21) Application number: 14810569.5
(22) Date of filing: 11.06.2014
(51) Int. Cl.: H01L 51/50, C09K 11/06, C07D 491/147, C07D 491/048, C07D 519/00

(54) **MATERIAL FOR ORGANIC ELECTROLUMINESCENT ELEMENTS, ORGANIC ELECTROLUMINESCENT ELEMENT USING SAME, AND ELECTRONIC DEVICE**
MATERIAL FÜR ORGANISCHE ELEKTROLUMINESZIERENDE ELEMENTE, ORGANISCHES ELEKTROLUMINESZIERENDES ELEMENT DAMIT UND ELEKTRONISCHE VORRICHTUNG
MATIÈRE POUR ÉLÉMENTS ÉLECTROLUMINESCENTS ORGANIQUES, ÉLÉMENT ÉLECTROLUMINESCENT ORGANIQUE UTILISANT CETTE MATIÈRE ET DISPOSITIF ÉLECTRONIQUE

(30) Priority: 11.06.2013 JP 2013122806
(43) Date of publication of application: 20.04.2016
(73) Proprietor: Idemitsu Kosan Co., Ltd, Tokyo 100-8321 (JP)
(72) Inventor: HAYAMA, Tomoharu, Sodegaura-shi Chiba 299-0293 (JP); KAWAMURA, Masahiro, Sodegaura-shi Chiba 299-0293 (JP); MIZUKI, Yumiko, Sodegaura-shi Chiba 299-0293 (JP); ITO, Hirokatsu, Sodegaura-shi Chiba 299-0293 (JP); HAKETA, Tasuku, Sodegaura-shi Chiba 299-0293 (JP)
(74) Representative: Hoffmann Eitle
(86) International application number: PCT/JP2014/003116
(87) International publication number: WO 2014/199637

(56) References cited:
- WO-A1-95/04059
- WO-A1-2010/137285
- WO-A1-2011/137157
- WO-A1-2012/070226
- WO-A1-2012/090970
- WO-A1-2012/102967
- WO-A1-2013/056776
- WO-A2-2005/009398
- JP-A- 2008 074 939
- JP-A- 2011 084 531
- JP-A- 2013 232 520
- JP-A- 2013 234 183
- KR-A- 20120 104 067
- US-A1- 2013 099 208
- US-A1- 2014 034 915

## Description

### Technical Field

The invention relates to a material for an organic electroluminescence device comprising an azanaphthobenzofuran derivative, an organic electroluminescence device using the same and an electronic apparatus.

### Background Art

In general, an organic electroluminescence (EL) device comprises an anode, a cathode and one or more organic thin film layers disposed between the anode and the cathode. When a voltage is applied between the both electrodes, to the emitting region, electrons are injected from the cathode and holes are injected from the anode. These injected electrons and holes are re-combined in the emitting region, create an excited state, and energy is emitted as light when the excited state is returned to the ground state.

Since an organic EL device can emit various emission colors by using various emitting materials in the emitting layer, practical application thereof to a display or the like has been actively studied. In particular, researches on emitting materials of the three primary colors of red, green and blue are conducted most actively, and extensive studies have been made in order to attain improvement in properties.

For example, as the material for an organic EL device, a compound having a naphthobenzofuran structure is disclosed in Patent Documents 1 to 3. Further, Patent Document 4 discloses use of a compound having a dibenzofuran structure or a dinaphthofuran structure as the material for an organic EL device. In the field of an organic EL device, development of a series of new materials is required in order to attain further improvement in device performance.

### Related Art Documents

### Patent Documents

Patent Document 1: WO2010/036027
Patent Document 2: WO2010/137285
Patent Document 3: WO2011/137157
Patent Document 4: WO2006/128800

### Summary of the Invention

An object of the invention is to provide a novel material that is effective as a material for an organic EL device.

According to one aspect of the invention, a material for an organic electroluminescence device comprising a compound represented by the following formula (2-1') as defined in claim 1 is provided.

According to the invention, a novel material that is effective as a material for an organic EL device can be provided.

### Brief Description of the Drawings

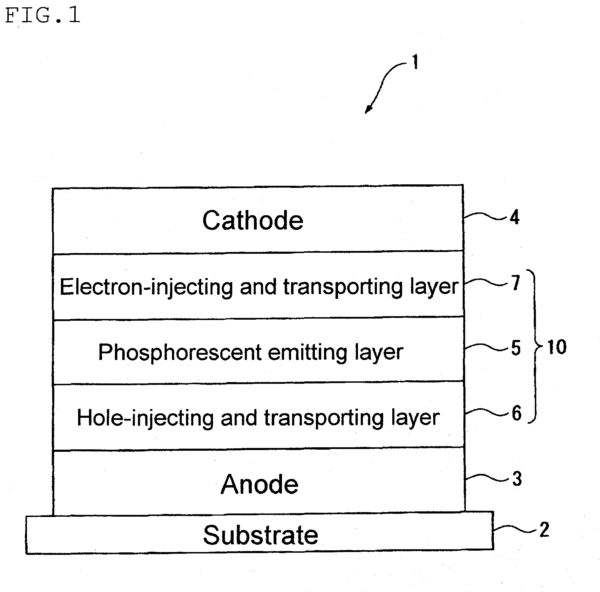

FIG. 1 is a schematic cross-sectional view showing one example of the organic EL device of the invention.

### Mode for Carrying out the Invention

In the invention, the "a to b" carbon atoms" in the "a substituted or unsubstituted X group including a to b carbon atoms" means the number of carbon atoms in the X group which is unsubstituted, and does not include the number of carbon atoms in a substituent when the X group is substituted by the substituent.

In the invention, the "hydrogen atom" includes isotopes differing in number of neutrons, i.e. protium, deuterium and tritium.

Further, the arbitrary substituent in the "substituted or unsubstituted" is one selected from the group consisting of an alkyl group including 1 to 50 (preferably1 to 18, more preferably 1 to 8) carbon atoms; a cycloalkyl group including 3 to 50 (preferably 3 to 10, more preferably 3 to 8, further preferably 5 or 6) carbon atoms that form a ring (hereinafter referred to as the "ring carbon atoms"); an aryl group including 6 to 50 (preferably 6 to 25, more preferably 6 to 18) ring carbon atoms; an aralkyl group including 7 to 51 (preferably 7 to 30, more preferably 7 to 20) carbon atoms having an aryl group including 6 to 50 (preferably 6 to 25, more preferably 6 to 18) ring carbon atoms; an amino group; a mono-substituted or di-substituted amino group having a substituent selected from an alkyl group including 1 to 50 (preferably1 to 18, more preferably 1 to 8) carbon atoms and an aryl group including 6 to 50 (preferably 6 to 25, more preferably 6 to 18) ring carbon atoms; an alkoxy group including 1 to 50 (preferably 1 to 18, more preferably 1 to 8) carbon atoms; an aryloxy group having an aryl group including 6 to 50 (preferably 6 to 25, more preferably 6 to 18) ring carbon atoms; a mono-substituted, di-substituted or tri-substituted silyl group having a substituent selected from an alkyl group including 1 to 50 (preferably 1 to 18, more preferably 1 to 8) carbon atoms and an aryl group including 6 to 50 (preferably 6 to 25, more preferably 6 to 18) carbon atoms; a heteroaryl group including 5 to 50 (preferably 5 to 24, more preferably 5 to 13) atoms that form a ring (hereinafter referred to as the "ring atoms"); a haloalkyl group including 1 to 50 (preferably 1 to 18, more preferably 1 to 8) carbon atoms; a halogen atom (fluorine atom, chlorine atom, bromine atom, iodine atom); a cyano group; a nitoro group; a sulfonyl group having a substituent selected from an alkyl group including 1 to 50 (preferably 1 to 18, more preferably 1 to 8) carbon atoms and an aryl group including 6 to 50 (preferably 6 to 25, more preferably 6 to 18) ring carbon atoms; a di-substituted phosphoryl group including 1 to 50 (preferably 1 to 18, more preferably1 to 8) carbon atoms and an aryl group including 6 to 50 (preferably 6 to 25, more preferably 6 to 18) ring carbon atoms; an alkylsulfonyloxy group; an arylsulfonyloxy group; an alkylcarbonyloxy group; an arylcarbonyloxy group; a boron-containing group; a zinc-containing group; a tin-containing group; a silicon-containing group; a magnesium-containing group; a lithium-containing group; a hydroxy group; an alkyl-substituted or aryl-substituted carbonyl group; a carboxy group; a vinyl group; a (meth)acryloyl group, an epoxy group; and an oxetanyl group.

These substituents may further be substituted by the above-mentioned arbitrary substituent.

### [Material for an organic electroluminescence device]

The material for an organic electroluminescence material as one embodiment of the invention is characterized in that it comprises a compound represented by any of the following formula (2-1') as defined in claim 1:

In the formulas (1) to (3), the substituents represented by R₁ are independently selected from the following group (A), preferably the following group (B), and more preferably the following group (C).

The above-mentioned group (A) is a group consisting of a substituted or unsubstituted alkyl group including 1 to 50 carbon atoms; a substituted or unsubstituted cycloalkyl group including 3 to 50 ring carbon atoms; a substituted or unsubstituted aryl group including 6 to 50 ring carbon atoms; a substituted or unsubstituted aralkyl group including 7 to 51 carbon atoms; an amino group; a mono-substituted or di-substituted amino group having a substituent selected from a substituted or unsubstituted alkyl group including 1 to 50 carbon atoms or a substituted or unsubstituted aryl group including 6 to 50 ring carbon atoms; a mono-substituted, di-substituted or tri-substituted silyl group having a substituent selected from a substituted or unsubstituted alkoxyl group including 1 to 50 carbon atoms, a substituted or unsubstituted aryloxy group including 6 to 50 ring carbon atoms, a substituted or unsubstituted alkyl group including 1 to 50 carbon atoms and a substituted or unsubstituted aryl group including 6 to 50 ring carbon atoms; a substituted or unsubstituted heteroaryl group including 5 to 50 ring atoms, a substituted or unsubstituted haloalkyl group including 1 to 50 carbon atoms; a halogen atom; a cyano group; a nitro group; a sulfonyl group having a substituent selected from a substituted or unsubstituted alkyl group including 1 to 50 carbon atoms and a substituted or unsubstituted aryl group including 6 to 50 ring carbon atoms; a di-substituted phosphoryl group having a substituent selected from a substituted or unsubstituted alkyl group including 1 to 50 carbon atoms, a substituted or unsubstituted heteroaryl group including 5 to 50 ring atoms and a substituted or unsubstituted aryl group including 6 to 50 ring carbon atoms; an alkylsufonyloxy group; an arylsulfonyloxy group; an alkylcarbonyloxy group; an arylcarbonyloxy group; a boron-containing group; a zinc-containing group; a tin-containing group; a silicon-containing group; a magnesium-containing group; a lithium-containing group; a hydroxy group; an alkyl-substituted or aryl-substituted carbonyl group; a carboxy group; a vinyl group; a (meth)acryloyl group; an epoxy group; and an oxetanyl group.

The above-mentioned group (B) is a group consisting of a substituted or unsubstituted alkyl group including 1 to 50 carbon atoms; a substituted or unsubstituted cycloalkyl group including 3 to 50 ring carbon atoms; a substituted or unsubstituted aryl group 6 to 50 ring carbon atoms; a substituted or unsubstituted aralkyl group including 7 to 51 carbon atoms; an amino group; a mono-substituted or di-substituted amino group having a substituent selected from a substituted or unsubstituted alkyl group including 1 to 50 carbon atoms and a substituted or unsubstituted aryl group including 6 to 50 ring carbon atoms; a mono-substituted, di-substituted or tri-substituted silyl group having a substituent selected from a substituted or unsubstituted alkoxy group including 1 to 50 carbon atoms, a substituted or unsubstituted aryloxy group including 6 to 50 ring carbon atoms, a substituted or unsubstituted alkyl group including 1 to 50 carbon atoms and a substituted or unsubstituted aryl group including 6 to 50 ring carbon atoms; a substituted or unsubstituted heteroarayl group including 5 to 50 ring atoms, a substituted or unsubstituted haloalkyl group 1 to 50 carbon atoms; a halogen atom; a cyano group; a nitro group; a sulfonly group having a substituent selected from a substituted or unsubstituted alkyl group including 1 to 50 carbon atoms and a substituted or unsubstituted aryl group including 6 to 50 ring carbon atoms; and a substituted or unsubstituted alkyl group including 1 to 50 carbon atoms and a substituted or unsubstituted aryl group including 6 to 50 ring carbon atoms.

The above-mentioned group (C) is a group consisting of a substituted or unsubstituted alkyl group including 1 to 50 carbon atoms; a substituted or unsubstituted cycloalkyl group including 3 to 50 ring carbon atoms; a substituted or unsubstituted aryl group including 6 to 50 ring carbon atoms; a substituted or unsubstituted aralkyl group including 7 to 51 carbon atoms; an amino group; a mono-substituted or di-substituted amino group having a substituent selected from a substituted or unsubstituted alkyl group including 1 to 50 carbon atoms and a substituted or unsubstituted aryl group including 6 to 50 ring carbon atoms; a mono-substituted, di-substituted or tri-substituted silyl group having a substituent selected from a substituted or unsubstituted alkoxy group including 1 to 50 carbon atoms, a substituted or unsubstituted aryloxy group including 6 to 50 ring carbon atoms, a substituted or unsubstituted alkyl group including 1 to 50 carbon atoms and a substituted or unsubstituted aryl group including 6 to 50 ring carbon atoms; a substituted or unsubstituted heteroaryl group including 5 to 50 ring atoms, a substituted or unsubstituted haloalkyl group including 1 to 50 carbon atoms; a halogen atom; a cyano group; and a nitro group.

R₂ is a substituted or unsubstituted alkyl group including 1 to 50 carbon atoms, a substituted or unsubstituted haloalkyl group including 1 to 50 carbon atoms, a cyano group or a halogen atom.

Specific examples of R₁, R₂, R₁₁ (mentioned later) or the like will be given.

As the alkyl group including 1 to 50 (preferably 1 to 18, more preferably 1 to 8) carbon atoms, for example, methyl, ethyl, n-propyl, isopropyl, n-butyl, isobutyl, s-butyl, t-butyl, pentyl (including isomers), hexyl (including isomers), heptyl (including isomers), octyl (including isomers), nonyl (including isomers), decyl (including isomers), undecyl (including isomers), and dodecyl (including isomers), tridecyl, tetradecyl, octadecyl, tetracosanyl, tetracontanyl or the like can be given. Among these, methyl, ethyl, n-propyl, isopropyl, n-butyl, isobutyl, s-butyl, t-butyl, pentyl (including isomers), hexyl (including isomers), heptyl (including isomers), octyl (including isomers), nonyl (including isomers), decyl (including isomers), undecyl (inlcuding isomers), dodecyl (isomers), tridecyl, tetradecyl and octadecyl are preferable. Methyl, ethyl, n-propyl, isopropyl, n-butyl, isobutyl, s-butyl, t-butyl, pentyl (including isomers), hexyl (including isomers), heptyl (including isomers) and octyl (including isomers) are more preferable.

As the cycloalkyl group including 3 to 50 (preferably 3 to 10, more preferably 3 to 8, and further preferably 5 or 6) ring carbon atoms, a cyclopropyl group, a cyclobutyl group, a cyclopentyl group, a cyclohexyl group, a cycloheptyl group, a cyclooctyl group, an adamantyl group or the like can be given. Among these, a cyclopentyl group and a cyclohexyl group are preferable.

As the aryl group including 6 to 50 (preferably 6 to 25, more preferably 6 to18) ring carbon atoms, for example, phenyl, naphthyl, naphthylphenyl, biphenylyl, terphenylyl, acenaphthylenyl, anthryl, benzoanthryl, aseanthryl, phenanthryl, benzophenanthryl, phenalenyl, fluorenyl, 9,9'-spirobifluorenyl, benzofluorenyl, dibenzofluorenyl, picenyl, pentaphenyl, pentacenyl, pyrenyl, chrysenyl, benzochrysenyl, s-indacenyl, as-indacenyl, fluoranthenyl, benzofluoranthenyl, tetracenyl, triphenylenyl, benzotriphenylenyl, perylenyl, coronyl, dibenzoanthryl or the like can be given.

As the arylene group including 6 to 50 (preferably 6 to 25, more preferably 6 to 18) ring carbon atoms, one obtained by removing a hydrogen atom from the above-mentioned aryl group can be given.

The heteroaryl group including 5 to 50 (preferably 5 to 24, more preferably 5 to 13) ring atoms contains at least one, preferably 1 to 5 (more preferably 1 to 3, further preferably 1 to 2) hetero atoms (e.g. a nitrogen atom, a sulfur atom, an oxygen atom, a phosphorus atom). As the heteroaryl group, for example, a pyrrolyl group, a furyl group, a thienyl group, a pyridyl group, a pridazinyl group, a pyrimidinyl group, a pyrazinyl group, a triazinyl group, an imidazolyl group, an oxazolyl group, a thiazolyl group, a pyrazolyl group, an isoxazolyl group, an isothiazolyl group, an oxadiazolyl group, a thiadiazolyl group, a triazolyl group, a tetrazolyl group, an indolyl group, an isoindolyl group, a benzofuranyl group, an isobenzofuranyl group, a benzothiophenyl group, an isobenzothiophenyl group, an indolizinyl group, a quinolizinyl group, a quinolyl group, an isoquinolyl group, a cinnolyl group, a phthalazinyl group, a quinazolinyl group, a quinoxalinyl group, a benzimidazolyl group, a benzoxazolyl group, a benzothiazolyl group, an indazolyl group, a benzisoxazolyl group, a benzisothiazolyl group, a dibenzofuranyl group, a dibenzothiophenyl group, a carbazolyl group, a phenanthridinyl group, an acridinyl group, a phenanthrolinyl group, a phenazinyl group, a phenothiazinyl group, a phenoxazinyl group, an azatriphenylenyl group, a diazatriphenylenyl group, a xanthenyl group, an azacarbazolyl group, an azadibenzofuranyl group, an azadibenzothiophenyl group, a benzofuranobenzothiophenyl group, a benzothienobenzothiophenyl group, a dibenzofuranonaphthyl group, a dibenzothienonaphthyl group and a dinaphthothienothiophenyl group or the like can be given.

As specific examples of the heteroaryl group including 5 to 50 ring atoms, a monovalent group obtained by removing one hydrogen atom from the compounds represented by the following formulas is preferable. 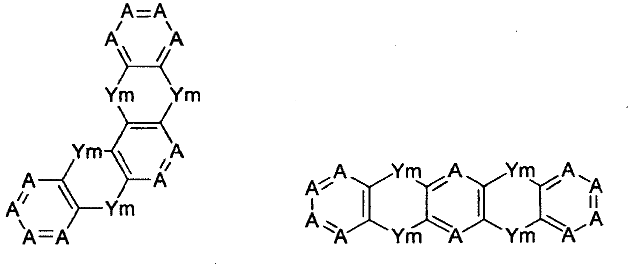 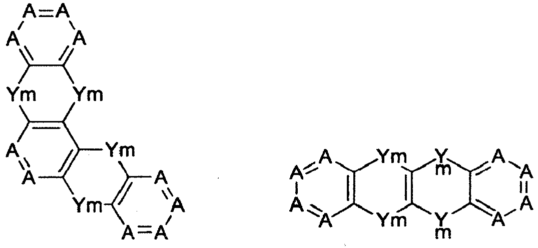 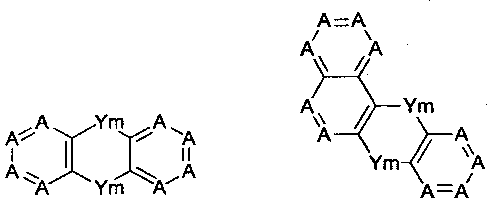 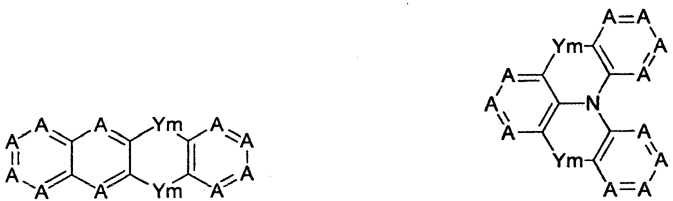 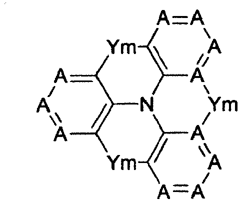 wherein in the formulas, As are independently CR²⁰⁰ or a nitrogen atom and R²⁰⁰s are independently a hydrogen atom or a substituent,
Ys are independently a single bond, C(R²⁰¹)(R²⁰²), an oxygen atom, a sulfur atom or N(R²⁰³); and
R²⁰¹, R²⁰² and R²⁰³ are independently a hydrogen atom or a substituent, and ms are independently 0 or 1.
In the formula, plural As may be the same as or different from each other, plural Ys may be the same as or different from each other, and plural ms may be the same as or different from each other.

When m is 0, no Y is present.

As the substituent in the formula, the same as those mentioned above can be given.

As the aralkyl group including 7 to 51 carbon atoms in total having an aryl group including 6 to 50 (preferably 6 to 25, more preferably 6 to 18) ring carbon atoms, an aralkyl group having the aryl group mentioned above can be given.

As the mono-substituted or di-substituted amino group having a substituent selected from an alkyl group including 1 to 50 (preferably 1 to 18, more preferably 1 to 8) carbon atoms and an aryl group including 6 to 50 (preferably 6 to 25, more preferably 6 to 18) ring carbon atoms, and a mono-substituted or di-substituted amino group having a substituent selected from the alkyl group or the aryl group mentioned above can be given.

As the alkoxy group having the above-mentioned alkyl group including 1 to 50 (preferably 1 to 18, more preferably 1 to 8) carbon atoms, the alkoxy group having the alkyl group mentioned above can be given.

As the aryloxy group having the above-mentioned aryl group including 6 to 50 (preferably 6 to 25, more preferably 6 to 18) ring carbon atoms, an aryloxy group having the aryl group mentioned above can be given.

As the mono-substituted, di-substituted or tri-substituted silyl group having a substituent selected from an alkyl group including 1 to 50 (preferably 1 to 18, more preferably 1 to 8) carbon atoms and an aryl group having 6 to 50 (preferably 6 to 25, more preferably 6 to 18) ring carbon atoms, a mono-substituted, di-substituted or tri-substituted silyl group having a substituent seleted from the alkyl group and the aryl group can be given.

As the above-mentioned haloalkyl group including 1 to 50 (preferably 1 to 18, more preferably 1 to 8) carbon atoms, one in which one or more hydrogen atoms of the alkyl group is substituted by a halogen atom (a fluorine atom, a chlorine atom, a bromine atom, an iodine atom) can be given.

As the sulfonyl group having a substituent selected from the above-mentioned alkyl group including 1 to 50 (preferably1 to 18, more preferably 1 to 8) carbon atoms and the aryl group having a substituent including 6 to 50 (preferably 6 to 25, more preferably 6 to 18) ring carbon atoms, a sulfonyl group having a substituent selected from the above-mentioned akyl group or the above-mentioned aryl group can be given.

As the di-substituted phosphoryl group having an alkyl group including 1 to 50 (preferably 1 to 18, more preferably 1 to 8) carbon atoms and the aryl group including 6 to 50 (preferably 6 to 25, more preferably 6 to 18) ring carbon atoms, a di-substituted phosphoryl group having a substituent selected from the alkyl group and the aryl group can be given.

In the formula, the ring structure formed by bonding of the adjacent substituents may be a saturated ring or an unsaturated ring.

As the saturated ring, independently, an aliphatic hydrocarbon ring having 3 to 50 (preferably 3 to 6, more preferably 5 or 6) ring carbon atoms is preferable.

As the unsaturated ring, independently, an aromatic hydrocarbon ring including 6 to 50 (preferably 6 to 24, more preferably 6 to 18) ring carbon atoms or an aromatic heterocyclic ring including 5 to 50 (preferably 5 to 24, more preferably 5 to 13) ring atoms is preferable.

As specific examples of the aliphatic hydrocarbon ring including 3 to 50 ring carbon atoms, a cyclopropane ring, a cyclobutane ring, a cyclopentane ring, a cyclohexane ring, a cycloheptane ring, a cyclooctane ring, an adamantane ring can be given. Among these, a cyclopentane ring and a cyclohexane ring are preferable.

As specific examples of the aromatic hydrocarbon ring including 6 to 50 ring carbon atoms, a benzene ring, a naphthalene ring, an anthracene ring, a benzoanthracene ring, a phenanthrene ring, a benzophenanthrene ring, a fluorene ring, a benzofluorene ring, a dibenzofluorene ring, a picene ring, a tetracene ring, a pentacene ring, a pyrene ring, a chrysene ring, a benzochrysene ring, a s-indacene ring, an as-indacene ring, a fluoranthene ring, a benzofluoranthene ring, a triphenylene ring, a benzotriphenylene ring, a perylene ring, a coronene ring, a dibenzoanthracene ring can be given.

As specific examples of the above-mentioned aromatic heterocyclic ring including 5 to 50 ring atoms, a pyrrole ring, a pyrazole ring, an isoindole ring, a benzofuran ring, a benzothiophene ring, an isobenzofuran ring, a dibenzothiophene ring, an isoquinoline ring, a cinnoline ring, a quinoxaline ring, a phenanthridine ring, a phenanthroline ring, a pyridine ring, a pyrazine ring, a pyrimidine ring, a pyridazine ring, a triazine ring, an imidazopyridine ring, an indole ring, an indazole ring, a benzimidazole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzoxazole ring, a thiazole ring, a thiadiazole ring, a benzothiazole ring, a triazole ring, an imidazole ring, a benzimidazole ring, a pyran ring, a dibenzofuran ring, a benzo[c]dibenzofuran ring, a purine ring, and an acridine ring can be given.

As the aromatic hydrocarbon group including 6 to 60 ring carbon atoms, the aryl group including 6 to 50 ring carbon atoms mentioned above as the examples of the substituent can be given.

As the heterocyclic group including 3 to 60 ring atoms, the heteroaryl group including 5 to 50 ring atoms mentioned above as the examples of the substituent can be given.

In R₂₀ examples of the aryl group and the heteroaryl group are the same as those for R₁ mentioned above.

Examples of the compound represented by the formula (2-1') will be given below.

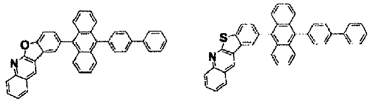

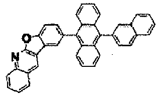

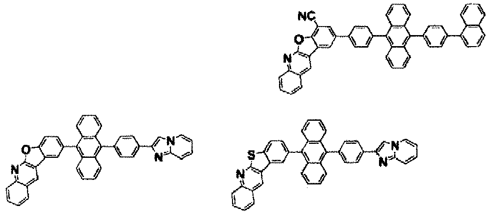

The material for an organic EL device of the invention comprises the compound represented by the above formula (2-1'). The content of the above-mentioned compound in the material for an organic EL device is not particularly restricted. For example, it may be 1 mass% or more, preferably 10 mass% or more, more preferably 50 mass% or more, further preferably 80 mass% or more, and particularly preferably 90 mass% or more. The content may be 100 mass%. As other materials than those represented by the formula (2-1'), materials used in the emiting layer, the electron-transporting layer, the hole-transporting layer or the like (mentioned later) can be given.

The material for an organic EL device of the invention is effective as the material for an organic EL device, and can be used as a host material or a dopant material in the emitting layer of a fluorescent emitting unit or as a host material in the emitting layer of a phosphorescent emitting unit. In any of a fluorescent emitting unit and a phosphorescent emitting unit, the material is effective as a material for an anode-side organic thin film layer provided between an anode and an emitting layer of an organic EL device or as a material for a cathode-side organic thin film layer provided between a cathode and an emitting layer of an organic EL device. That is, it is effective as a material for a hole-transporting layer, a hole-injecting layer, an electron-transporting layer, an electron-injecting layer, a hole-blocking layer, an electron-blocking layer, or the like.

Meanwhile, the "emitting unit" means the minimum unit that comprises one or more organic layers, one of which being an emitting layer, and can emit light by recombination of holes and electrons injected.

### [Organic EL device]

The organic EL device as one embodiment of the invention comprises one or more organic thin film layers including an emitting layer between a cathode and an anode, and at least one layer of the organic thin film layers comprises the above-mentioned material for an organic EL device.

As examples of the organic thin film layers that comprise the above-mentioned material for an organic EL device, an anode-side organic thin film layer (hole-transporting layer, hole-injecting layer, or the like), an emitting layer, a cathode-side organic thin film layer (electron-transporting layer, electron-injecting layer, or the like) provided between a cathode and an emitting layer, a spacing layer, a barrier layer or the like can be given. The examples are not limited thereto. The above-mentioned material for an organic EL device may be contained in any of the above-mentioned layers, and can be used as a host material or a dopant material in the emitting layer of a fluoresecent emitting unit, a host material in the emitting layer of a phosophorescent emitting unit, a hole-transporting layer, an electron-transporting layer or the like of an emitting unit.

The organic EL device of the invention may be a fluorescent or phosphorescent monochromatic emitting device or may be a fluorescent/phosphorescent hybride white emitting device. It may be a simple emitting device having a single emitting unit or a tandem emitting device having plural emitting units. Among them, the organic EL device may preferably be a phospresecent emitting device.

As the representative device structure of a simple type organic EL device, the following device configuration can be given.

### (1) Anode/emitting unit/cathode

The emitting unit mentioned above may be a stacked type emitting unit comprising plural phorphosrecent emitting layers or plural fluorescent emitting layers. In this case, in order to prevent diffusion of excitons generated in the phosphorescent emitting layer to the fluorescent emitting layer, a spacing layer may be provided between the emtitting layers. The representative layer configuration of the emitting unit is given below.
(a) Hole-transporting layer/Emitting layer (/Electron-transporting layer)
(b) Hole-transporting layer/First phosphorescent emitting layer/Second phosphorescent emitting layer (/Electron-transporting layer)
(c) Hole-transporting layer/Phosphorecent emitting layer/Spacing layer/Fluorecent emitting layer (/Electron-transporting layer)
(d) Hole-transporting layer/First phosphorescent emitting layer/Second phosphorescent emitting layer/Spacing layer/Fluorecent emitting layer (/Electron-transporting layer)
(e) Hole-transporting layer/First phosphorescent emitting layer/Spacing layer/Second phosphorescent emitting layer/Spacing layer/Fluorecent emitting layer (/Electron-transporting layer)
(f) Hole-transporting layer/Phosphorecent emitting layer/Spacing layer/First fluorecent emitting layer/Second fluorecent emitting layer (/Electron-transporting layer)
(g) Hole-transporting layer/Electron barrier layer/Emitting layer (/Electron-transporting layer)
(h) Hole-transporting layer/Emitting layer/Hole barrier layer (/Electron-transporting layer)
(i) Hole-transporting layer/Fluorecent emitting layer/Triplet barrier layer (/Electron-transporting layer)

The phosphorescent or fluorecent emitting layer as mentioned above can emit different colors of light. Specifically, in the stacked emitting layer (d), a layer configuration of the hole-transporting layer/first phosphorescent emitting layer (red emission)/second phosphorescent emitting layer (green emission)/spacing layer/fluorescent emitting layer (blue emission)/electron-transporting layer or the like can be given.

Between each emitting layer and the hole-transporting layer or the spacing layer, an electron-barrier layer may be provided appropriately. Between each emitting layer and the electron-transporting layer, a hole-barrier layer may be provided appropriately. Due to provision of an electron-barrier layer or a hole-barrier layer, electrons or holes can be confined within the emitting layer, whereby possibility of recombination of carriers in the emitting layer can be increased, and the life can be improved.

As the represented device configuration of a tandem organic EL device, the following device configuration can be given.

### (2) Anode/first emitting unit/intermediate layer/second emitting unit/cathode

Here, as the first emitting unit and the second emitting unit, the same emitting units as those mentioned above can independently be given, for example.

In general, the intermediate layer is called an intermediate electrode, an intermediate conductive layer, a carrier-generating layer, an electron-withdrawing layer, and a known material configuration that supplies electrons to the first emitting unit and supplies holes to the second emitting unit can be used.

FIG. 1 shows a schematic configuration of one example of the organic EL device of the invention. The organic EL device 1 comprises a substrate 2, an anode 3, a cathode 4 and an emitting unit 10 provided between the anode 3 and the cathode 4. The emitting unit 10 comprises an emitting layer 5 that includes at least one phosphorescent emitting layer comprising a phosphorescent host material and a phosphorescent dopant. A hole-injecting and transporting layer 6 or the like may be provided between the emitting layer 5 and the anode 3 and an electron-injecting and transporting layer 7 or the like may be provided between the emitting layer 5 and the cathode 4. An electron-barrier layer may be provided on the anode 3 side of the emitting layer 5 and a hole-barrier layer may be provided on the cathode 4 side of the emitting layer 5. Due to such configuration, electrons or holes can be confined in the emitting layer 5, whereby possibility of generation of excitons in the emitting layer 5 can be improved.

Herein, a host that is combined with a fluorescent dopant is referred to as a fluorescent host and a host that is combined with a phosphorescent dopant is referred to as a phosphorescent host. The fluorescent host and the phosphorescent hoast are not distinguished only by the molecular structure thereof. That is, the phosphorescent host means a material constituting a phosphorescent emitting layer that contains a phosphorescent dopant and does not mean a material that cannot be used as a material constituting a fluorescent dopant. The same can be applied to a fluorescent host.

### (Substrate)

The organic EL device is usually formed on a transparent substrate. The transparent substrate is a substrate for supporting the organic EL device, and is preferably a flat and smooth substrate having a 400-to-700-nm-visible-light transmittance of 50% or more. Specific examples thereof include glass plates and polymer plates. Examples of the glass plate include those obtained by using as raw materials soda-lime glass, barium/strontium-containing glass, lead glass, aluminosilicate glass, borosilicate glass, barium borosilicate glass, quartz, or the like. Examples of the polymer plate include those obtained by using as raw materials polycarbonate, acrylic polymer, polyethylene terephthalate, polyethersulfide, polysulfone, or the like.

### (Anode)

The anode of the organic EL device plays a role for injecting holes into its hole-transporting layer or emitting layer. It is effective to use one having a work function of 4.5 eV or more. As specific examples of the anode material, indium tin oxide alloy (ITO), tin oxide (NESA), indium zinc oxide, gold, silver, platinum, copper, and the like can be given. The anode can be formed by forming these electrode materials into a thin film by vapor deposition, sputtering or the like. In the case where emission from the emitting layer is taken out through the anode, the transmittance of the anode to the emission is preferably more than 10%. The sheet resistance of the anode is preferably several hundred Ω/□ or less. The film thickness of the anode, which varies depending upon the material thereof, is usually from 10 nm to 1 µm, preferably from 10 to 200 nm.

### (Cathode)

The cathode plays a role for injecting electrons into its electron-injecting layer, electron-transporting layer or emitting layer. The cathode is preferably formed of a material having a small work function. The cathode material is not particulary restricted. As specific examples of the cathode material, indium, aluminum, magnesium, a magnesium-indium alloy, a magnesium-aluminum alloy, an aluminum-lithium alloy, an aluminum-scandium-lithium alloy, a magnesium-silver alloy or the like can be given: As in the case of the anode, the cathode can be formed by forming the materials into a thin film by a deposition method, a sputtering method or the like. If necessary, emission can be outcoupled from the cathode side.

### (Emitting layer)

The emitting layer is an organic layer having an emitting function, and where a doping system is used, it comprises a host material and a dopant material. The host material has a function of accelerating recombination of electrons and holes and confining excitons within the emitting layer. The dopant material has a function of emitting efficiently excitons obtained by recombination.

In the case of a phosphorescent device, the host material has a function of confining excitons mainly generated by a dopant within the emitting layer.

Here, in the emitting layer, a double host (also referred to as a host/cohost) that adjusts the carrier balance in the emitting layer may be used by combining an electron-transporting host and a hole-transporting host or by other methods. It is preferred that the emitting layer comprise a first host material and a second host material and that the first host material be the material for the organic EL device according to the invention.

Double dopant may be used in which two or more types of dopant materials having a high quantum yield are incorporated, and each dopant emits light. Specifically, by allowing a host, a red dopant and a green dopant to be co-deposited, yellow emission from the common emitting layer, whereby yellow emission is realized.

As for the emitting layer, by allowing plural emitting layers to be a stacked body, electrons and holes are accumulated in the interface of the emitting layers, whereby the recombination region is concentrated in the interface of the emitting layers. As a result, the quantum efficiency is improved.

Easiness in injection of holes to the emitting layer and easiness in injection of electrons to the emitting layer may differ. Further, the hole-transporting performance and the electron-transporting performance indicated by the mobility of holes and electrons in the emitting layer may differ from each other.

The emitting layer can be formed by a known method such as a deposition method, a spin coating method, a LB method (Langmuir Blodgett method) or the like, for example. The emitting layer can also be formed by forming a solution obtained by dissolving a binder such as a resin and material compounds in a solvent into a thin film by a spin coating method and the like.

The emitting layer is preferably a molecular deposited film. The "molecular deposited film" means a thin film formed by deposition of a raw material compound in a vapor phase or a film formed by solidification of a raw material compound in a solution state or a liquid phase state. Normally, this molecular deposited film differs from a thin film (molecular accumulated film) formed by a LB method in aggregation structure or high-order structure, or differ in function derived from such difference in structure.

The dopant material is selected from a known fluorescent dopant showing fluorescent emission or a known phosphorescent dopant showing phosphorescent emission.

A phosphorescent dopant (phosphorescent emitting material) that forms the emitting layer is a compound that can emit light from triplet excited state. The phosphorescent dopant is not limited as long as it can emit from triplet excited state. The phosphorescent dopant is preferably an organic metal complex containing at least one metal selected from Ir, Pt, Os, Au, Cu, Re and Ru and a ligand. It is preferred that the ligand have an ortho-metalated bond. In respect of a high phosphorescent quantum yield and capability of improving external quantum yield of an emitting device, the phosphorescent dopant is preferably a compound having a metal atom selected from Ir, Os and Pt. Further preferable are a metal complex such as an iridium complex, an osmium complex and a platinum complex, with an ortho-metalated complex being more preferable. Among them, an iridium complex and a platinum complex are more preferable, and an ortho-metalated iridium complex is particularly preferable.

The content of the phosphorescent dopant in the emitting layer is not particularly restricted, and it may be appropriately selected depending on the purpose. For example, the content is preferably 0.1 to 70 mass%, with 1 to 30 mass% being more preferable. When the content of the phosphorescent compound is 0.1 mass% or more, sufficient emission can be obtained. By allowing the content to be 70 mass% or less, it is possible to suppress a phenomenon called concentration quenching.

Specific examples of an organic metal complex that is preferably as a phosphorescent dopant are shown below.

An abbrivation under the specific examples, i.e. PQIr(iridium(lll)bis(2-phenylquinolyl-N,C^{2'})acetylacetonate) and Ir(ppy)₃(tris(2-phenylpyridinate-N,C2') iridum (III)), is an abbreviation of an organic metal complex shown above the abbreviation.

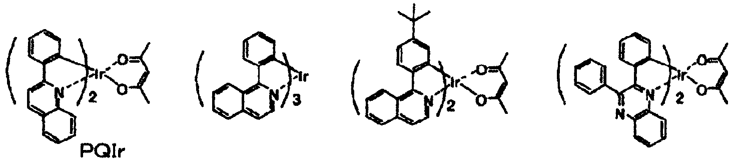

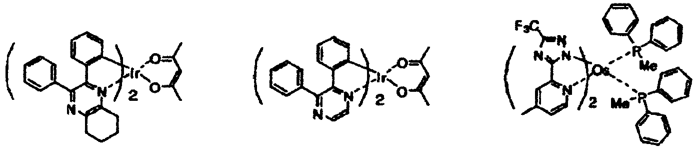

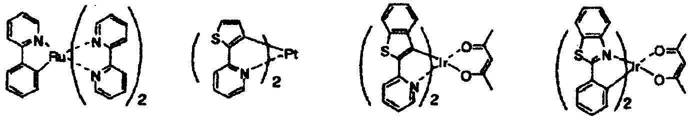

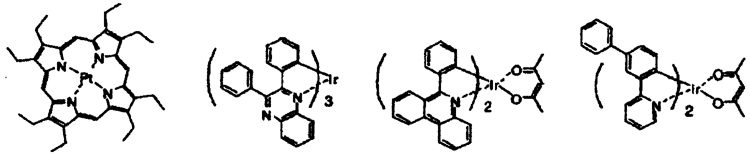

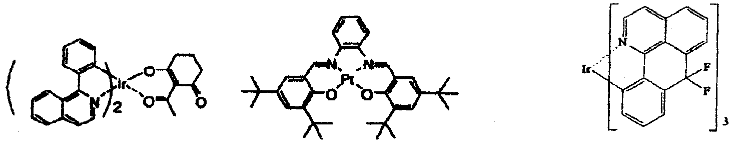

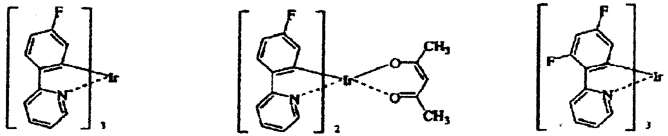

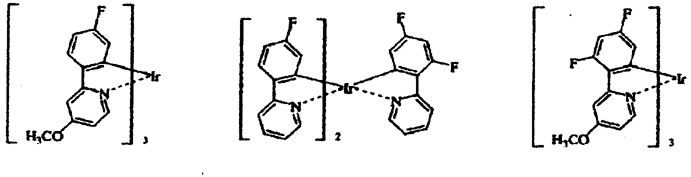

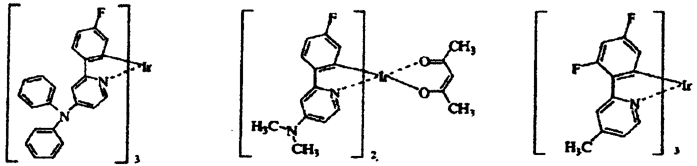

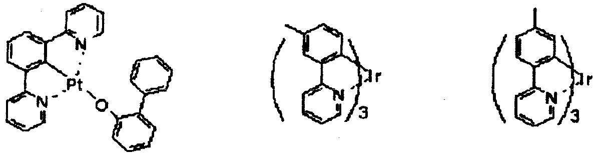

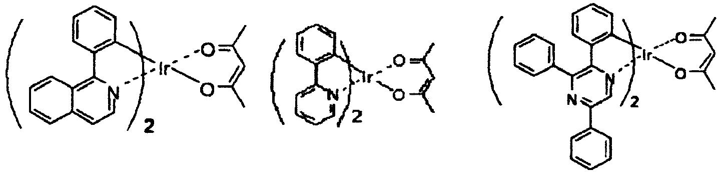

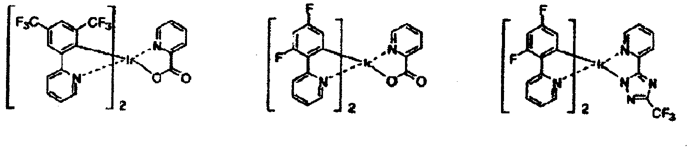

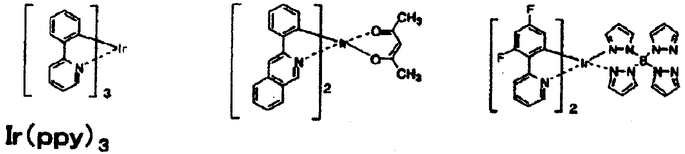

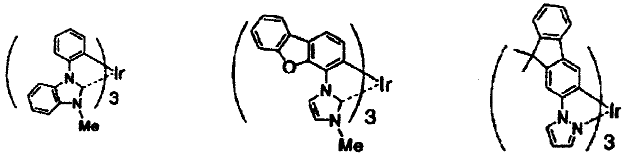

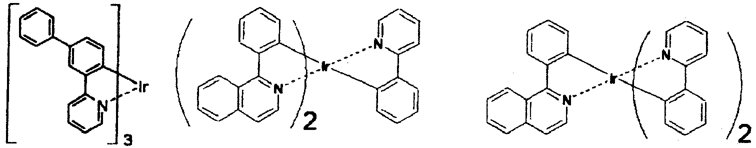

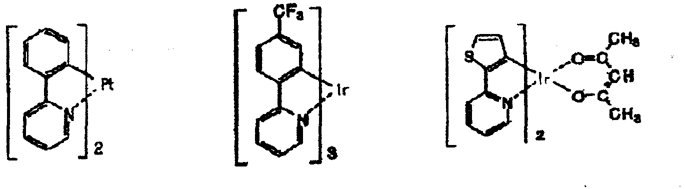

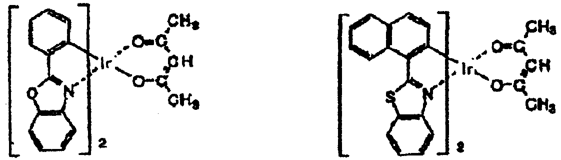

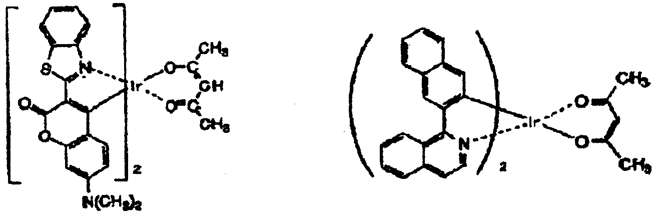

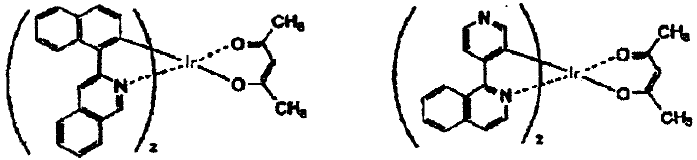

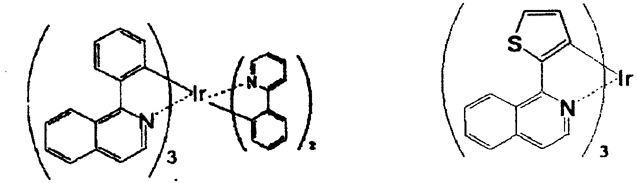

The phosphorescent host is a compound having a function of allowing a phosphorescent dopant to emit light efficiently by efficiently confining the triplet energy of the phosphorescent dopant in the emitting layer. The material for an organic EL device according to the invention is preferable as the phosphorescent host. The emitting layer may comprise one kind of the material for an organic EL device according to the invention or may comprise two or more kinds of the material for an organic EL device according to the invention.

When the material for an organic EL device according to the invention is used as a host material of the emitting layer, the emission wavelength of the phosphorescent dopant contained in the emitting layer is not particularly restricted. It is preferred that at least one kind of the phosphorescent dopant materials contained in the emitting layer have a peak of an emission wavelength of 490 nm or more and 700 nm or less, more preferably 490 nm or more and 650 nm or less. As for the emission color of the emitting layer, red, yellow and green are preferable, for example. By using the compound according to the invention as the host material and by forming an emitting layer by doping the phosphorescent dopant having such an emission wavelength, it is possible to obtain a long-lived organic EL device.

In the organic EL device according to the invention, other compounds than the material for an organic EL device according to the invention can appropriately be selected as the phosphorescent host according to the above-mentioned purpose.

The material for an organic EL device according to the invention and other compounds may be used in combination as the phosphorescent host material in the same emitting layer. When plural emitting layers are present, as the phosphorescent host material for one of these emitting layers, the material for an organic EL device according to the invention is used, and as the phosphorescent host material for one of other emitting layers, other compounds than the material for an organic EL device according to the invention may be used. The material for an organic EL device according to the invention can be used in an organic layer other than the emitting layer. In that case, as the phosphorescent host of the emitting layer, other compounds than the material for an organic EL device according to the invention may be used.

As for the compound other than the material for an organic EL device according to the invention, as specific examples of the compound that is preferable as the phosphorescent host, carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidene-based compounds, porphyrin-based compounds, anthraquinodimethane derivatives, anthrone derivatives, diphenylquinone derivatives, thiopyrandioxide derivatives, carbodiimide derivatives, fluorenylidene methane derivatives, distyrylpyrazine derivatives and heterocyclic tetracarboxylic anhydrides of naphthaleneperylene or the like, metal complexes of phthalocyanine derivatives and 8-quinolinol derivatives, various metal complex polysilane compounds represented by metal complexes having metal phthalocyanine, benzoxazole or benzothiazole as a ligand, poly(N-vinylcarbazole) derivatives, aniline-based copolymers, conductive polymer oligomers such as thiophene oligomers and polythiophene, and polymer compounds such as polythiophene derivatives, polyphenylene derivatives, polyphenylene vinylene derivatives and polyfluorene derivatives can be given. The phosphorescent host may be used alone or in combination of two or more. As specific examples, the following compounds can be given.

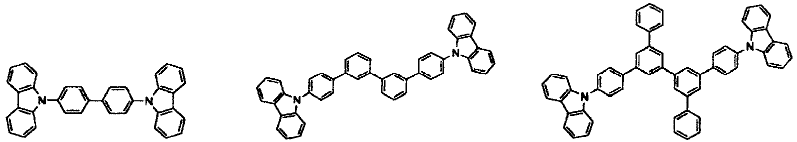

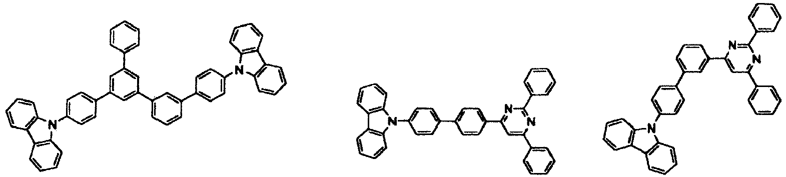

If the emitting layer comprises the first host material and the second host material, the material for an organic EL device according to the invention may be used as the first host material and other compounds than the material for an organic EL device according to the invention may be used as the second host material. The "first host material" and the "second host material" as referred to herein mean that the plural host materials contained in the emitting layer differ from each other in structure, and are not determined by the content of each host material in the emitting layer.

The second host material is not particularly restricted, and compounds other than the material for an organic EL device according to the invention and the same compound mentioned above as being preferable as the phosphorescent host can be given. As the second host, a carbazole derivative, an arylamine derivative, a fluorenone derivative and an aromatic tertiary amine compound are preferable.

The organic EL device of the invention may have an emitting layer that contains a fluorecent emitting material (i.e. fluorecent emitting layer). As the fluorecent emitting layer, a known fluorecent emitting material can be used. As the fluorecent emitting material, at least one selected from an anthracene derivative, a fluororanthene derivative, a styrylamine derivative and an arylamine derivative is preferable. An anthracene derivative and an arylamine derivative are more preferable. In particular, an anthracene derivative is preferable as a host material, and an arylamine derivative is preferable as a dopant. Specifically, preferable materials disclosed in WO2010/134350 or WO2010/134352 can be selected. The material for an organic EL device of the invention may be used as a fluorecent emitting material for the fluorecent emitting layer, or may be used as a host material for the fluorecent emitting layer.

The ring carbon atoms of the anthracene derivative as a fluorecent emitting layer is preferably 26 to 100, more preferably 26 to 80, and further preferably 26 to 60. As the anthracene derivative, more specifically, an anthracne derivative represented by the following formula (10) is preferable.

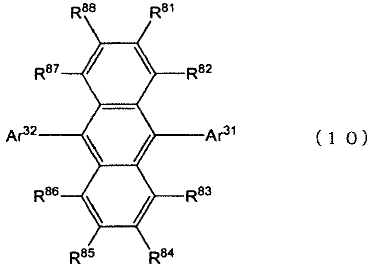

In the formula (10), Ar³¹ and Ar³² are independently a substituted or unsubstituted aryl group including 6 to 50 ring carbon atoms or a heterocyclic group including 5 to 50 ring atoms.

R⁸¹ to R⁸⁸ are independently a hydrogen atom, a substituted or unsubstituted aryl group including 6 to 50 ring carbon atoms, a substituted or unsubstituted heterocyclic group including 5 to 50 ring atoms, an alkyl group including 1 to 50 carbon atoms, a substituted or unsubstituted alkoxy group including 1 to 50 carbon atoms, a substituted or unsubstituted aralkyl group including 7 to 50 carbon atoms, a substituted or unsubstiuted aryloxy group including 6 to 50 ring carbon atoms, a substituted or unsubstituted arylthio group including 6 to 50 ring carbon atoms, a substituted or unsubstituted alkoxycarbonyl group including 2 to 50 carbon atoms, a substituted or unsubstituted silyl group, a carboxy group, a halogen atom, a cyano group, a nitro group or a hydroxyl group.

As the above-mentioned any aryl group including 6 to 50 ring carbon atoms, an aryl group including 6 to 40 ring carbon atoms is preferable, with an aryl group including 6 to 30 ring carbon atoms being more preferable.

As the above-mentioned any heterocyclic group including 5 to 50 ring atoms, a heterocyclic group including 5 to 40 ring atoms is preferable, with a heterocyclic group including 5 to 30 ring atoms being more preferable.

As the above-mentioned alkyl group including 1 to 50 carbon atoms, an alkyl group including 1 to 30 carbon atoms is preferable, an alkyl group including 1 to 10 carbon atoms are more preferable, with an alkyl group including 1 to 5 carbon atoms being further preferable.

As the above-mentioned alkoxy group including 1 to 50 carbon atoms, an alkoxy group including 1 to 30 carbon atoms is preferable, an alkoxy group including 1 to 10 carbon atoms is more preferable, with an alkoxy group including 1 to 5 carbon atoms being further preferable.

As the above-mentioned aralkyl group including 7 to 50 carbon atoms, an aralkyl group including 7 to 30 carbon atoms is preferable, with an aralkyl group including 7 to 20 carbon atoms being more preferable.

As the above-mentioned aryloxy group including 6 to 50 ring carbon atoms, an aryloxy group including 6 to 40 ring carbon atoms is preferable, with an aryloxy group including 6 to 30 ring carbon atoms being more preferable.

As the above-mentioned arylthio group including 6 to 50 ring carbon atoms, an arylthio group including 6 to 40 ring carbon atoms is preferable, with an arylthio group including 6 to 30 ring carbon atoms being more preferable.

As the above-mentioned alkoxycarbonyl group including 2 to 50 carbon atoms, an alkoxycarbonyl group including 2 to 30 carbon atoms is preferable, an alkoxycarbonyl group including 2 to 10 carbon atoms is more preferable, with an alkoxycarbonyl group including 2 to 5 carbon atoms being further preferable.

As the above-mentioned halogen atom, a fluorine atoms, a chlorine atom, a bromine atom or the like may be given.

In particular, Ar³¹ and Ar³² are preferably a substituted or unsubstituted aryl group including 6 to 50 ring carbon atoms.

As the anthracene derivative represented by the formula (10), an anthracene derivative represetend by the following formula (10-1) is preferable.

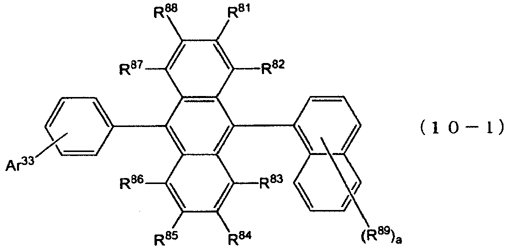

In the formula (10-1), Ar³³ is a substituted or unsubstituted aryl group including 6 to 50 ring carbon atoms or a heterocyclic group including 5 to 50 ring atoms. R⁸¹ to R⁸⁸ are as defined above. R⁸⁹ is the same as defined for R⁸¹ to R⁸⁸. a is an integer of 1 to 7.

Preferable examples of R⁸¹ to R⁸⁸ are the same as defined above. Preferable examples of R⁸⁹ are the same as those for R⁸¹ to R⁸⁸. a is preferably an integer of 1 to 3, with 1 or 2 being more preferable.

As the aryl group including 6 to 50 ring carbon atoms represented by Ar3³, an aryl group including 6 to 40 ring carbon atoms is preferable, an aryl group including 6 to 30 ring carbon atoms is more preferable, an aryl group including 6 to 20 ring carbon atoms is further preferable, with an aryl group including 6 to 12 ring carbon atoms being particularly preferable.

As the arylamine derivative as the fluorescent emitting material, an aryldiamine derivative is preferable, an aryldiamine derivative having a pyrene skeleton is more preferable, and an aryldiamine derivative having a pyrene skeleton and a dibenzofurane skeleton is further preferable.

As the aryldiamine derivative, more specifically, the arylamine derivative represented by the following formula (11) is preferable.

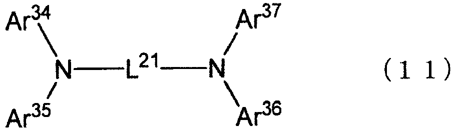

In the formula (11), Ar³⁴ to Ar³⁷ are independently a substituted or unsubstituted aryl group including 6 to 50 ring carbon atoms or a substituted or unsubstituted heteroaryl group including 5 to 50 ring atoms.

L²¹ is a substituted or unsubstituted arylene group including 6 to 50 ring carbon atoms or a substituted or unsubstituted heteroarylene group including 5 to 50 ring atoms.

As the aryl group including 6 to 50 ring carbon atoms, an aryl group including 6 to 30 ring carbon atoms is preferable, an aryl group including 6 to 20 ring carbon atoms is more preferable, an aryl group including 6 to 12 ring carbon atoms is further preferable, with a phenyl group and a naphthyl group being particularly preferable.

As the heteroaryl group including 5 to 50 ring atoms, a heteroaryl group including 5 to 40 ring atoms is preferable, a heteroaryl group including 5 to 30 ring atoms is more preferable and a heteroaryl group including 5 to 20 ring atoms are further preferable. As the heteroaryl group, a carbazolyl group, a dibenzofuranyl group, a dibenzofuranyl group or the like can be given, and a dibenzofuranyl group is preferable. As the preferable substitutent of the heteroaryl group, an aryl group including 6 to 30 (preferably 6 to 20, more preferably 6 to12) ring carbon atoms can be given, with a phenyl group and a naphthyl group being more preferable.

As the arylene group including 6 to 50 ring carbon atoms, an arylene group including 6 to 40 ring carbon atoms is preferable, an arylene group including 6 to 30 ring carbon atoms is more preferable, an arylene group including 6 to 20 ring carbon atoms is further preferable, with a pyrenyl group being particularly preferable.

The thickness of the emitting layer is preferably 5 to 50 nm, more preferably 7 to 50 nm, and further preferably 10 to 50 nm. If the thickness is 5 nm or more, the formation of the emitting layer is facilitated. If the thickness is 50 nm or less, an increase in driving voltage can be avoided.

### (Electron-donating dopant)

In the organic EL device according to the invention, it is preferred that an electron-donating dopant be contained in the interfacial region between the cathode and the emitting unit. Due to such a configuration, the organic EL device can have an increased luminance or a long life. Here, the electron-donating dopant means one having a metal with a work function of 3.8 eV or less. As specific examples thereof, at least one selected from an alkali metal, an alkali metal complex, an alkali metal compound, an alkaline earth metal, an alkaline earth metal complex, an alkaline earth metal compound, a rare earth metal, a rare earth metal complex and a rare earth metal compound or the like can be mentioned.

As the alkali metal, Na (work function: 2.36 eV), K (work function: 2.28 eV), Rb (work function: 2.16 eV), Cs (work function: 1.95 eV) and the like can be given. One having a work function of 2.9 eV or less is particularly preferable. Among them, K, Rb and Cs are preferable. Rb or Cs is further preferable. Cs is most preferable. As the alkaline earth metal, Ca (work function: 2.9 eV), Sr (work function: 2.0 eV to 2.5 eV), Ba (work function: 2.52 eV) and the like can be given. One having a work function of 2.9 eV or less is particularly preferable. As the rare-earth metal, Sc, Y, Ce, Tb, Yb and the like can be given. One having a work function of 2.9 eV or less is particularly preferable.

Examples of the alkali metal compound include an alkali oxide such as Li₂O, Cs₂O or K₂O, and an alkali halide such as LiF, NaF, CsF and KF. Among them, LiF, Li₂O and NaF are preferable. Examples of the alkalineearth metal compound include BaO, SrO, CaO, and mixtures thereof such as BaₓSr₁₋ₓO (0<x<1) and BaₓCa₁₋ₓO (0<x<1). Among them, BaO, SrO and CaO are preferable. Examples of the rare earth metal compound include YbF₃, ScF₃, ScO₃, Y₂O₃, Ce₂O₃, GdF₃ and TbF₃. Among these, YbF₃, ScF₃ and TbF₃ are preferable.

The alkali metal complexes, the alkaline earth metal complexes and the rare earth metal complexes are not particularly limited as long as they contain, as a metal ion, at least one of alkali metal ions, alkaline earth metal ions, and rare earth metal ions. Meanwhile, preferred examples of the ligand include, but are not limited to, quinolinol, benzoquinolinol, acridinol, phenanthridinol, hydroxyphenyloxazole, hydroxyphenylthiazole, hydroxydiaryloxadiazole, hydroxydiarylthiadiazole, hydroxyphenylpyridine, hydroxyphenylbenzimidazole, hydroxybenzotriazole, hydroxyfluborane, bipyridyl, phenanthroline, phthalocyanine, porphyrin, cyclopentadiene, β-diketones, azomethines, and derivatives thereof.

Regarding the addition form of the electron-donating dopant, it is preferred that the electron-donating dopant be formed in a shape of a layer or an island in the interfacial region. A preferred method for the formation is a method in which an organic compound (a light emitting material or an electron-injecting material) for forming the interfacial region is deposited simultaneously with deposition of the electron-donating dopant by a resistant heating deposition method, thereby dispersing the electron-donating dopant in the organic compound. The dispersion concentration of the organic compound : the electron-donating dopant (molar ratio) is 100:1 to 1:100, preferably 5:1 to 1:5.

In a case where the electron-donating dopant is formed into the shape of a layer, the light-emitting material or electron-injecting material which serves as an organic layer in the interface is formed into the shape of a layer. After that, a reductive dopant is solely deposited by the resistant heating deposition method to form a layer preferably having a thickness of from 0.1 nm to 15 nm. In a case where the electron-donating dopant is formed into the shape of an island, the emitting material or the electron-injecting material which serves as an organic layer in the interface is formed into the shape of an island. After that, the electron-donating dopant is solely deposited by the resistant heating deposition method to form an island preferably having a thickness of from 0.05 nm to 1 nm.

The ratio of the main component and the electron-donating dopant in the organic EL device according to the invention is main component: electron-donating dopant = 5 : 1 to 1 : 5 in terms of molar ratio, more preferably 2 : 1 to 1 : 2.

### (Electron-transporting layer)

The electron-transporting layer is an organic layer that is formed between the emitting layer and the cathode and has a function of transporting electrons from the cathode to the emitting layer. When the electron-transporting layer is formed of plural layers, an organic layer that is nearer to the cathode is often defined as the electron-injecting layer. The electron-injecting layer has a function of injecting electrons from the cathode efficiently to the organic layer unit. The material for an organic EL device of the invention is also preferable as an electron-transporting layer material that constitutes an electron-transporting layer.

As the electron-transporting material used in the electron-transporting layer other than the material for an organic EL device of the invention, an aromatic heterocyclic compound having one or more hetero atoms in the molecule may preferably be used. In particular, a nitrogen-containing ring derivative is preferable. As the nitrogen-containing ring derivative, an aromatic ring having a nitrogen-containing six-membered or five-membered ring skeleton or a fused aromatic ring compound having a nitrogen-containing six-membered or five-membered ring skeleton is preferable.

As the nitrogen-containing ring derivative, a nitrogen-containing ring metal chelate complex represented by the following formula (A) is preferable, for example.

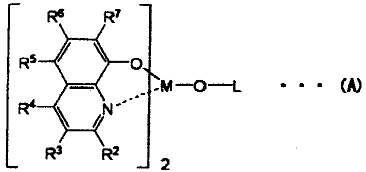

R² to R⁷ in the formula (A), that is a nitrogen-containing ring metal chelate complex, are independently a hydrogen atom, a heavy hydrogen atom, a hydrogen atom, a hydroxy group, an amino group, a hydrocarbon group including 1 to 40 carbon atoms, an alkoxy group including 1 to 40 carbon atoms, an aryloxy group including 6 to 50 carbon atoms, an alkoxycarbonyl group or an aromatic heterocyclic group including 5 to 50 ring carbon atoms. They may be substituted.

As the halogen atom, fluorine, chlorine, bromine, iodine or the like can be given, for example.

As examples of the amino group that may be substituted, an alkylamino group, an arylamino group and an aralkylamino group can be given.

The alkylamino group and the aralkylamino group are represented by -NQ¹Q². Q¹ and Q² are independently an alkyl group including 1 to 20 carbon atoms or an aralkyl group including 1 to 20 carbon atoms. One of Q¹ and Q² may be a hydrogen atom or a heavy hydrogen atom.

The arylamino group is represented by -NAr¹Ar², and Ar¹ and Ar² are independently a non-fused aromatic hydrocarbon group or fused aromatic hydrocarbon group including 6 to 50 carbon atoms. One of Ar¹ and Ar² may be either a hydrogen atom or a heavy hydrogen atom.

The hydrocarbon group including 1 to 40 carbon atoms includes an alkyl group, an alkenyl group, a cycloalkyl group, an aryl group and an aralkyl group.

The alkoxycarbonyl group is represented by -COOY' and Y' is an alkyl group including 1 to 20 carbon atoms.

M is aluminum (Al), gallium (Ga) or indium (In), and M is preferably In.

L is a group represented by the following formula (A') or (A").

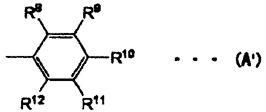

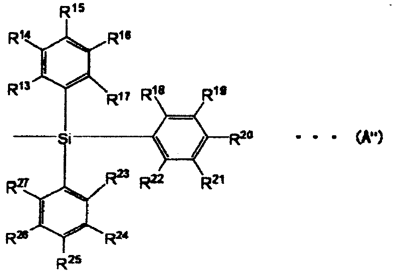

In the formula (A'), R⁸ to R¹² are independently a hydrogen atom, a heavy hydrogen atom or a substituted or unsubstituted hydrocarbon group including 1 to 40 carbon atoms, and adjacent groups may form a ring structure. In the formula (A"), R¹³ to R²⁷ are independently a hydrogen atom, a heavy hydrogen atom or a substituted or unsubstituted hydrocarbon group including 1 to 40 carbon atoms, and adjacent groups may form a ring structure.

The hydrocarbon group including 1 to 40 carbon atoms represented by R⁸ to R¹² and R¹³ to R²⁷ in the formulas (A') and (A") is the same as the hydrocarbon group represented by R² to R⁷ in the formula (A) that is a nitrogen-containing ring metal chelate complex. As the divalent group formed when the adjacent groups of R⁸ to R¹² and R¹³ to R²⁷ form a ring structure, a tetramethylene group, a pentamethylene group, a hexamethylene group, a diphenylmethane-2,2'-diyl group, a diphenylethane-3,3'-diyl group, a diphenylpropane-4,4'-diyl group or the like can be mentioned.

As the electron-transmitting material used in the electron-transmitting layer, a metal complex of 8-hydroxyquinoline or a derivative thereof, an oxadiazole derivative and a nitrogen-containing heterocyclic derivative are preferable. Specific examples of the metal complex of the 8-hydroxyquinoline or the derivative thereof include metal chelate oxynoid compounds containing a chelate of oxine (generally, 8-quinolinol or 8-hydroxyquinoline). For example, tris(8-quinolinol)aluminum can be used. As the oxadiazole derivative, the following can be given, for example.

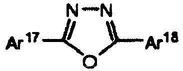

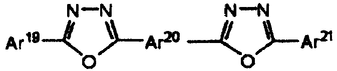

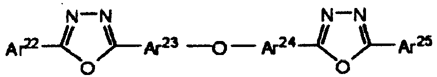

In the formula, Ar¹⁷, Ar¹⁸, Ar¹⁹, Ar²¹, Ar²² and Ar²⁵ are independently a substituted or unsubstituted aromatic hydrocarbon group or fused aromatic hydrocarbon group including 6 to 50 carbon atoms. Ar¹⁷ and Ar¹⁸, Ar¹⁹ and Ar²¹ and Ar²² and Ar²⁵ may be the same as or different from each other. As the aromatic hydrocarbon group or the fused aromatic hydrocarbon group, a phenyl group, a naphthyl group, a biphenyl group, an anthranyl group, a perylenyl group, a pyrenyl group or the like can be mentioned. As the substituent of these groups, an alkyl group including 1 to 10 carbon atoms, an alkoxy group including 1 to 10 carbon atoms, a cyano group or the like can be given.

Ar²⁰, Ar²³ and Ar²⁴ are independently a substituted or unsubstituted divalent aromatic hydrocarbon group or fused aromatic hydrocarbon group including 6 to 50 carbon atoms, and Ar²³ and Ar²⁴ may be the same as or different from each other. As the divalent aromatic hydrocarbon group or the fused aromatic hydrocarbon group, a phenylene group, a naphthylene group, a biphenylene group, an anthranylene group, a perylenylene group, a pyrenylene group or the like can be given. As the substituent of these, an alkyl group including 1 to 10 carbon atoms, an alkoxy group including 1 to 10 carbon atoms, a cyano group or the like can be given.

As these electron-transmitting compounds, those having excellent thin film-forming capability can be preferably used. As specific examples of these electron-transmitting compounds, the following can be given.

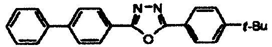

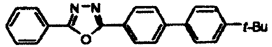

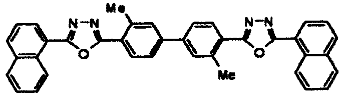

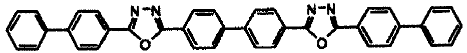

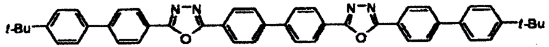

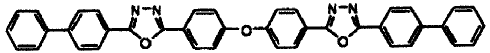

The nitrogen-containing heterocyclic derivative as the electron-transmitting compound is a nitrogen-containing heterocyclic derivative that comprises an organic compound represented by the following formula and is not a metal complex can be given. For example, a five-membered ring or a six-membered ring having a skeleton represented by the following formula (B) or one having a structure represented by the following formula (C) can be mentioned.

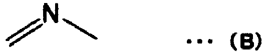

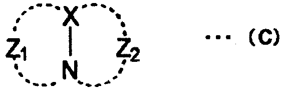

In the formula (C), X is a carbon atom or a nitrogen atom. Z₁ and Z₂ are independently a group of atoms capable of forming a nitrogen-containing heterocyclic ring.

The nitrogen-containing heterocyclic ring derivative is further preferably an organic compound having a nitrogen-containing aromatic polycyclic ring group composed of a five-membered ring or a six-membered ring. Further, in the case of the nitrogen-containing aromatic polycyclic ring group, a nitrogen-containing aromatic polycyclic organic compound having a skeleton obtained by combining the above formulas (B) and (C) or the above formula (B) and the following formula (D) is preferable.

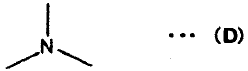

The nitrogen-containing group in the nitrogen-containing aromatic polycyclic organic compound can be selected from the nitrogen-containing heterocyclic groups represented by the following formulas, for example.

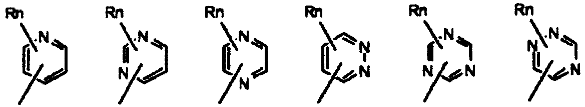

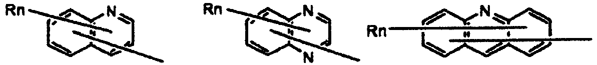

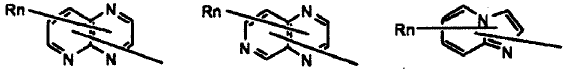

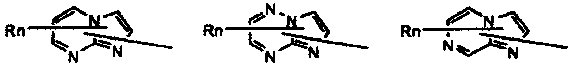

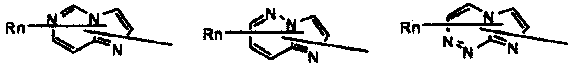

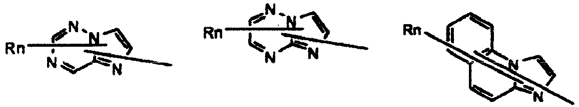

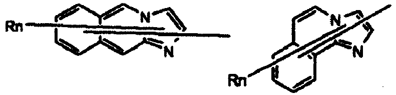

In each of the above formulas, R is an aromatic hydrocarbon group or fused aromatic hydrocarbon group including 6 to 40 carbon atoms, an aromatic heterocyclic group or fused aromatic heterocyclic group including 3 to 40 carbon atoms, an alkyl group including 1 to 20 carbon atoms or an alkoxy group including 1 to 20 carbon atoms. n is an integer of 0 to 5, and when n is an integer of 2 or more, plural Rs may be the same as or different from each other.

As further preferable specific compounds, a nitrogen-containing heterocyclic derivative represented by the following formula (D1) can be mentioned.

HAr-L¹-Ar¹-Ar² (D1)

In the formula (D1), HAr is a substituted or unsubstituted nitrogen-containing heterocyclic ring group including 3 to 40 carbon atoms, L¹ is a single bond, a substituted or unsubstituted aromatic hydrocarbon group or fused aromatic hydrocarbon group including 6 to 40 carbon atoms or a substituted or unsubstituted aromatic heterocyclic group or fused aromatic heterocyclic group including 3 to 40 carbon atoms, Ar¹ is a substituted or unsubstituted divalent aromatic hydrocarbon group including 6 to 40 carbon atoms, and Ar² is a substituted or unsubstituted aromatic hydrocarbon group or fused aromatic hydrocarbon group including 6 to 40 carbon atoms or a substituted or unsubstituted aromatic heterocyclic group or fused aromatic heterocyclic group including 3 to 40 carbon atoms.

HAr is selected from the following group, for example.

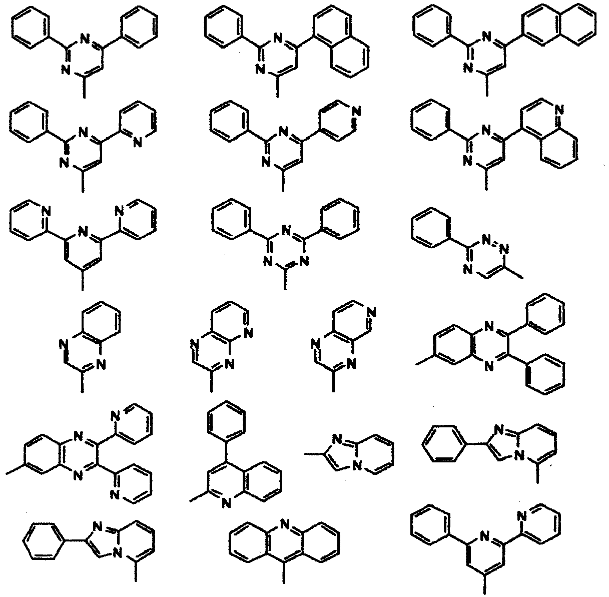

L¹ in the above formula (D1) is selected from the following group, for example.

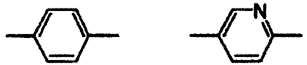

Ar¹ in the formula (D1) is selected from the arylanthranyl group in the following formulas (D2) and (D3).

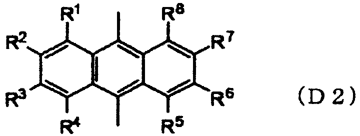

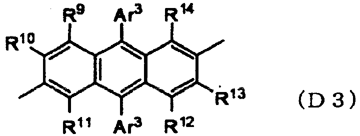

In the formulas (D2) and (D3), R¹ to R¹⁴ are independently a hydrogen atom, a heavy hydrogen atom, a halogen atom, an alkyl group including 1 to 20 carbon atoms, an alkoxy group including 1 to 20 carbon atoms, an aryloxy group including 6 to 40 carbon atoms, a substituted or unsubstituted aromatic hydrocarbon group or fused aromatic hydrocarbon group including 6 to 40 carbon atoms or a substituted or unsubstituted aromatic heterocyclic group or fused aromatic heterocyclic group including 3 to 40 carbon atoms; Ar³ is a substituted or unsubstituted aromatic hydrocarbon group or fused aromatic hydrocarbon group including 6 to 40 carbon atoms or a substituted or unsubstituted aromatic heterocyclic group or fused aromatic heterocyclic group including 3 to 40 carbon atoms. The nitrogen-containing heterocyclic derivative may be one in which all of R₁ to R⁸ are a hydrogen atom or a heavy hydrogen atom.

Ar² in the formula (D1) is selected from the following group, for example.

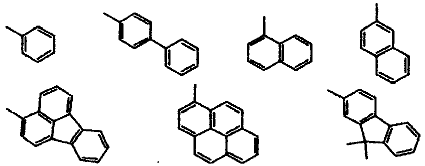

As the nitrogen-containing aromatic polycyclic organic compound as the electron-transmitting compound, in addition to those mentioned above, the following compounds can preferably be used.

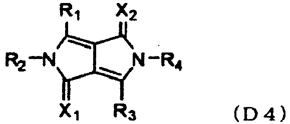

In the formula (D4), R₁ to R₄ are independently a hydrogen atom, a heavy hydrogen atom, a substituted or unsubstituted aliphatic group including 1 to 20 carbon atoms, a substituted or unsubstituted alicyclic group including 3 to 20 carbon atoms, a substituted or unsubstituted aromatic ring group including 6 to 50 carbon atoms or a substituted or unsubstituted heterocyclic group including 3 to 50 carbon atoms; and X₁ and X₂ are independently an oxygen atom, a sulfur atom or a dicyanomethylene group.

As the electron-transmitting compound, the following compound is preferably used.

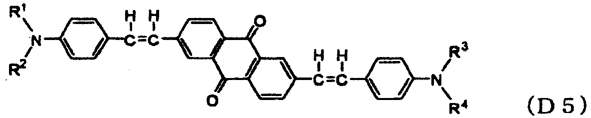

In the formula (D5), R¹, R², R³ and R⁴ are groups that are the same as or different from each other, and are an aromatic hydrocarbon group or a fused aromatic hydrocarbon group represented by the following formula (D6).

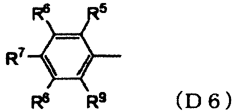

In the formula (D6), R⁵, R⁶, R⁷, R⁸ and R⁹ are groups that are the same as or different from each other, and are a hydrogen atom, a heavy hydrogen atom, a saturated or unsaturated alkoxy group including 1 to 20 carbon atoms, a saturated or unsaturated alkyl group including 1 to 20 carbon atoms, an amino group or an alkylamino group including 1 to 20 carbon atoms. At least one of R⁵, R⁶, R⁷, R⁸ and R⁹ is a group other than a hydrogen atom or a heavy hydrogen atom.

Further, the electron-transmitting compound may be a high molecular compound that comprises the nitrogen-containing heterocyclic group or the nitrogen-containing heterocyclic derivative.

It is particularly preferred that the electron-transporting layer of the organic EL device according to the invention contain at least one of the nitrogen-containing heterocyclic derivatives represented by the following formulas (E) to (G):

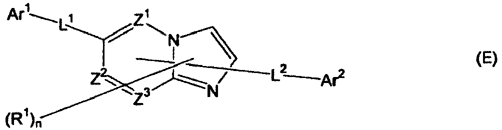

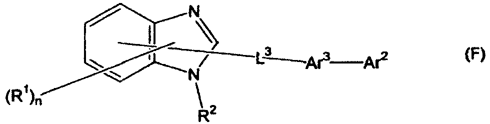

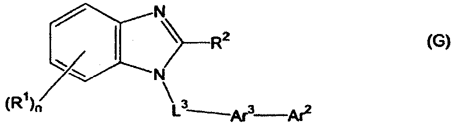

In the formulas (E) to (G), Z¹, Z² and Z³ are independently a nitrogen atom or a carbon atom.

R¹ and R² are independently a substituted or unsubstituted aryl group including 6 to 50 ring carbon atoms, a substituted or unsubstituted heteroaryl group including 5 to 50 ring atoms, a substituted or unsubstituted alkyl group including 1 to 20 carbon atoms, a substituted or unsubstituted haloalkyl group including 1 to 20 carbon atoms or a substituted or unsubstituted alkoxy group including 1 to 20 carbon atoms.

n is an integer of 0 to 5. When n is an integer of 2 or more, plural R¹s may be the same or different. The two adjacent R¹s may be bonded to each other to form a substituted or unsubstituted hydrocarbon ring.

Ar¹ is a substituted or unsubstituted aryl group including 6 to 50 ring carbon atoms or a substituted or unsubstituted heteroaryl group including 5 to 50 ring atoms.

Ar² is a hydrogen atom, a substituted or unsubstituted alkyl group including 1 to 20 carbon atoms, a substituted or unsubstituted haloalkyl group including 1 to 20 carbon atoms, a substituted or unsubstituted alkoxy group including 1 to 20 carbon atoms, a substituted or unsubstituted aryl group including 6 to 50 ring carbon atoms or a substituted or unsubstituted heteroaryl group including 5 to 50 ring atoms.

Any one of Ar¹ and Ar² is a substituted or unsubstituted fused aromatic hydrocarbon ring group including 10 to 50 ring carbon atoms or a substituted or unsubstituted fused aromatic heterocyclic group including 9 to 50 ring atoms.

Ar³ is a substituted or unsubstituted arylene group including 6 to 50 ring carbon atoms or a substituted or unsubstituted heteroarylene group including 5 to 50 ring atoms.

L¹, L² and L³ are independently a single bond, a substituted or unsubstituted arylene group including 6 to 50 ring carbon atoms or a substituted or unsubstituted divalent fused aromatic heterocyclic group including 9 to 50 ring atoms.

As the aryl group including 6 to 50 ring carbon atoms, a phenyl group, a naphthyl group, an anthryl group, a phenanthryl group, a naphthacenyl group, a chrysenyl group, a pyrenyl group, a biphenyl group, a terphenyl group, a tolyl group, a fluoranthenyl group and a fluorenyl group can be mentioned.

As the heteroaryl group including 5 to 50 ring atoms, a pyrrolyl group, a furyl group, a thienyl group, a silolyl group, a pyridyl group, a quinolyl group, an isoquinolyl group, a benzofuryl group, an imidazolyl group, a pyrimidyl group, a carbazolyl group, a selenophenyl group, an oxadiazolyl group, a triazolyl group, a pyrazinyl group, a pyridazinyl group, a triazinyl group, a quinoxalinyl group, an acridinyl group, an imidazo[1,2-a]pyridinyl group, an imidazo[1,2-a]pyrimidinyl group or the like can be given.

As the alkyl group including 1 to 20 carbon atoms, a methyl group, an ethyl group, a propyl group, a butyl group, a pentyl group, a hexyl group or the like can be given.

As the haloalkyl group including 1 to 20 carbon atoms, a group obtained by substituting one or two or more hydrogen atoms in the alkyl group with at least one halogen atom selected from fluorine, chlorine, iodine and bromine can be given.

As the alkoxy group including 1 to 20 carbon atoms, a group having the alkyl group as an alkyl moiety can be given.

As the arylene group including 6 to 50 ring carbon atoms, a group obtained by removing one hydrogen atom from the aryl group can be given.

As the divalent fused aromatic heterocyclic group including 9 to 50 ring atoms, a group obtained by removing one hydrogen atom from the fused aromatic heterocyclic group mentioned above as the heteroaryl group can be given.

The film thickness of the electron-transporting layer is not particularly restricted, but is preferably 1 nm to 100 nm.

As the constituting elements of the electron-injecting layer that can be provided in adjacent to the electron-transporting layer, in addition to the nitrogen-containing ring derivative, as an inorganic compound, it is preferable to use an insulator or a semiconductor. If the electron-injecting layer is formed of an insulator or a semiconductor, current leakage can be effectively prevented, whereby electron-injecting properties can be improved.

As such an insulator, it is preferable to use at least one metal compound selected from the group consisting of an alkali metal chalcogenide, an alkaline earth metal chalcogenide, a halide of an alkali metal and a halide of an alkaline earth metal. It is preferred that the electron-injecting layer be formed of these alkali metal chalcogenides or the like, since the electron-injecting property can be further improved. Specifically, as preferable alkali metal chalcogenides, Li₂O, K₂O, Na₂S, Na₂Se and Na₂O can be given. As preferable alkaline earth metal chalcogenides, CaO, BaO, SrO, BeO, BaS and CaSe can be given, for example. As preferable halides of an alkali metal, LiF, NaF, KF, LiCI, KCI, NaCl and the like can be given, for example. As preferable halides of an alkaline earth metal, a fluoride such as CaF₂, BaF₂, SrF₂, MgF₂ and BeF₂ and a halide other than a fluoride can be given, for example.

As the semiconductor, an oxide, a nitride or a nitric oxide containing at least one element selected from Ba, Ca, Sr, Yb, Al, Ga, In, Li, Na, Cd, Mg, Si, Ta, Sb and Zn or the like can be given, for example. They can be used singly or in combination of two or more. Further, it is preferred that an inorganic compound constituting the electron-injecting layer be a finely-crystallized or amorphous insulating thin film. If the electron-injecting layer is formed of these insulting thin films, more homogenous thin film is formed, and hence, pixel defects such as dark spots can be decreased. As such an inorganic compound, alkali metal chalcogenide, alkaline earth metal chalcogenide, a halide of an alkali metal and a halide of an alkaline earth metal or the like can be given, for example.

If such an insulator or a semiconductor is used, the preferable thickness of the layer is about 0.1 nm to 15 nm. The electron-injecting layer in the invention may preferably comprise the above-mentioned electron-donating dopant.

### (Hole-transporting layer)

The hole-transporting layer is an organic layer that is formed between the emitting layer and the anode, and has a function of transporting holes from the anode to the emitting layer. If the hole-transporting layer is composed of plural layers, an organic layer that is nearer to the anode may often be defined as the hole-injecting layer. The hole-injecting layer has a function of injecting holes efficiently to the organic layer unit from the anode.

As other materials that form the hole-transporting layer, an aromatic amine compound, for example, an aromatic amine derivative represented by the following formula (H) can preferably be used.

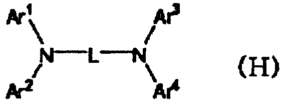

In the formula (H), Ar¹ to Ar⁴ are a substituted or unsubstituted aromatic hydrocarbon group or fused aromatic hydrocarbon group including 6 to 50 ring carbon atoms, a substituted or unsubstituted aromatic heterocyclic group or fused aromatic heterocyclic group including 5 to 50 ring atoms, or a group formed by bonding of these aromatic hydrocarbon group or the fused aromatic hydrocarbon group with an aromatic heterocyclic group or a fused aromatic heterocyclic group.

In the formula (H), L is a substituted or unsubstituted aromatic hydrocarbon group or fused aromatic hydrocarbon group including 6 to 50 ring carbon atoms or a substituted or unsubstituted aromatic heterocyclic group or fused aromatic heterocyclic group including 5 to 50 ring atoms.

Specific examples of the compound represented by the formula (H) are shown below.

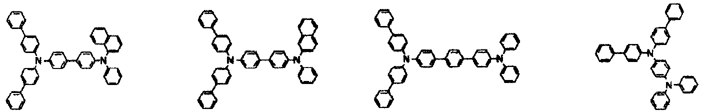

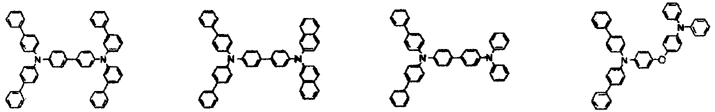

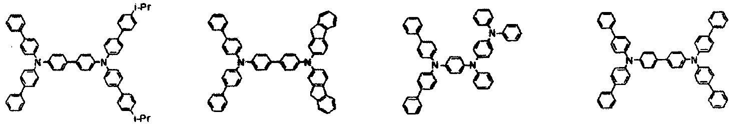

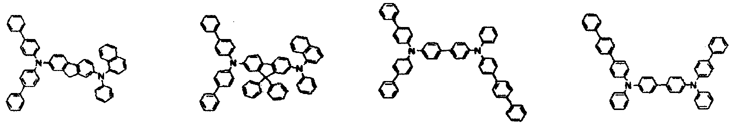

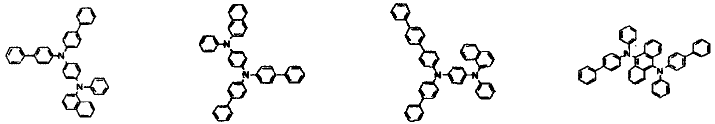

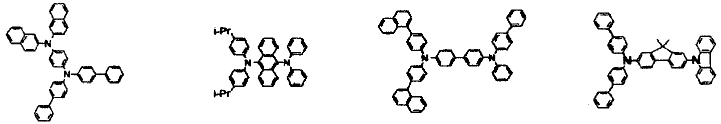

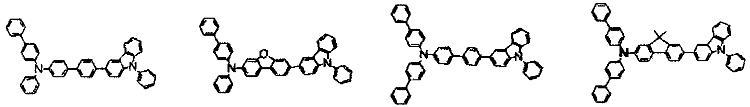

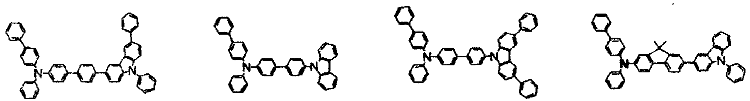

An aromatic amine represented by the following formula (J) is preferably used for forming the hole-transporting layer.

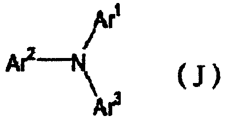

In the formula (J), Ar¹ to Ar³ are as defined for Ar¹ to Ar⁴ in the formula (H). Specific examples of the compound represented by the formula (J) will be shown below. The compound represented by the formula (J) is not limited to these.

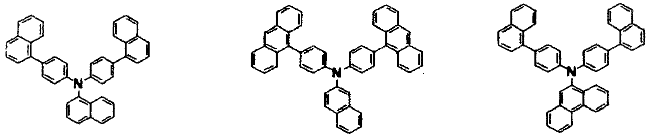

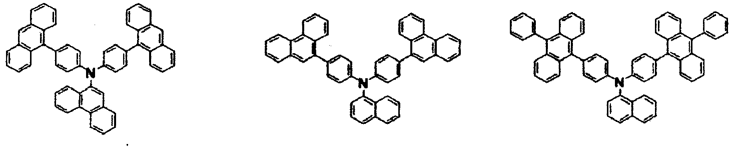

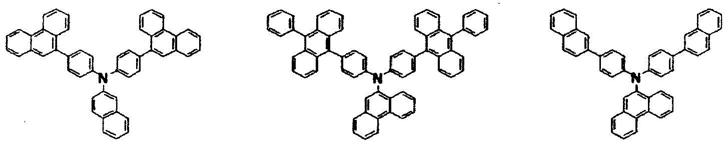

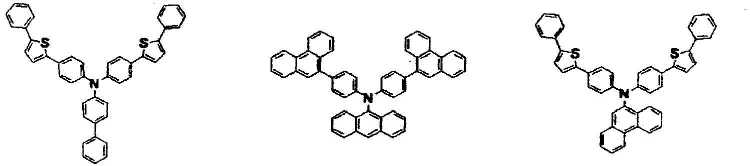

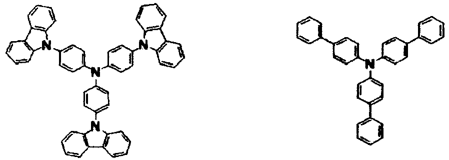

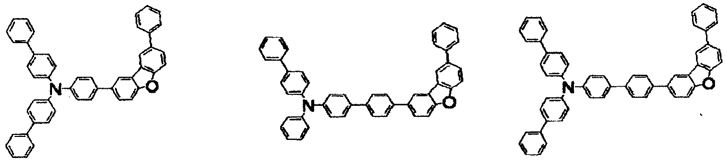

The hole-transporting layer of the organic EL device according to the invention may have a two-layer structure of a first hole-transporting layer (anode side) and a second hole-transporting layer (cathode side).

The thickness of the hole-transporting layer is not particularly restricted, but preferably 10 to 200 nm.

In the organic EL device according to the invention, a layer comprising an acceptor material may be stacked to the anode side of the hole-transporting layer or the first hole-transporting layer. As a result, a lowering in driving voltage or a decrease in production cost can be expected.

As the acceptor material, a compound represented by the following formula (K) is preferable.

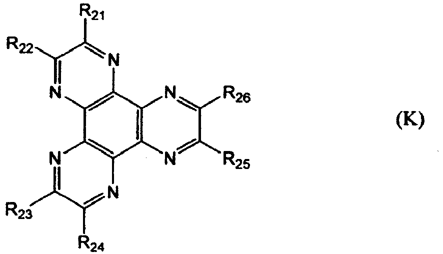

In the formula (K), R₂₁ to R₂₆, which may be the same as or different from each other, are independently a cyano group, ―CONH₂, a carboxyl group or ―COOR₂₇ (R₂₇ is an alkyl group including 1 to 20 carbon atoms or a cycloalkyl group including 3 to 20 carbon atoms); provided that, one or two or more pairs of R₂₁ and R₂₂; R₂₃ and R₂₄; and R₂₅ and R₂₆ may be bonded together to form a group represented by -CO-O―CO―.

As R₂₇, a methyl group, an ethyl group, an n-propyl group, an isopropyl group, an n-butyl group, an isobutyl group, a t-butyl group, a cyclopentyl group, a cyclohexyl group or the like can be given.

The thickness of the layer that comprises an acceptor material is not particularly limited, but preferably 5 to 20 nm.

### (n/p doping)

In the hole-transporting layer or the electron-transporting layer mentioned above, as described in the Japanese Patent No. 3695714, the carrier injecting performance can be adjusted by doping (n) of a donor material or doping (p) of an acceptor material.

As representative examples of the n-doping, a method in which an electron-transporting material is doped with a metal such as Li and Cs can be mentioned. As the represented example of the p-doping, a method in which a hole-transporting material is doped with an acceptor material such as F₄TCNQ (2,3,5,6-tetrafluoro-7,7,8,8-tetracyanoquinodimethane) can be given.

### (Spacing layer)

The spacing layer is a layer provided between the fluorescent emitting layer and the phosphorescent emitting layer when the fluorescent emitting layer and the phosphorescent emitting layer are stacked in order to prevent diffusion of excitons generated in the phosphorescent emitting layer to the fluorescent emitting layer or in order to adjust the carrier balance. Further, the spacing layer can be provided between the plural phosphorescent emitting layers.

Since the spacing layer is provided between the emitting layers, the material for the spacing layer is preferably a material having both electron-transporting properties and hole-transporting properties. In order to prevent diffusion of the triplet energy in adjacent phosphorescent emitting layers, it is preferred that the spacing layer have a triplet energy of 2.6 eV or more. As the material used for the spacing layer, the same material as those used in the above-mentioned hole-transporting layer can be given.

### (Barrier layer)

It is preferred that the organic EL device according to the invention have a barrier layer such as an electron-barrier layer, a hole-barrier layer and a triplet barrier layer in a part that is adjacent to the emitting layer. Here, the electron-barrier layer is a layer that serves to prevent leakage of electrons from the emitting layer to the hole-transporting layer, and the hole-barrier layer is a layer that serves to prevent leakage of holes from the emitting layer to the electron-transporting layer.

The triplet barrier layer prevents diffusion of triplet excitons generated in the emitting layer to the surrounding layers, and has a function of preventing energy deactivation of triplet excitons on molecules in the electron-transporting layer other than the emitting dopant by confining the triplet excitons within the emitting layer.

When the triplet barrier layer is provided, in the phosphorescent emitting device, the following is considered. The the triplet energy of the phosphorescent emitting dopant is taken as E^{T}_{d} and the triplet energy of the compound used as the triplet barrier layer is taken as E^{T}_{TB}. If the energy relationship E^{T}_{d} < E^{T}_{TB} is satisfied, in respect of energy, the triplet excitons of the phosphorescent emitting dopant is confined (i.e. the triplet excitons cannot be moved to other molecules), whereby the energy deactivation route other than emission on the dopant is cut off, leading to efficient emission. However, even when the relationship E^{T}_{d} < E^{T}_{TB} is established, if the energy difference ΔE^{T} = E^{T}_{TB} - E^{T}_{d} is small, it is thought that, in an environment at around room temperature where the device is actually driven, due to thermal energy of the surrounding area, the triplet excitons can move to other molecules by endothermically overcoming this energy difference ΔE^{T}. In particular, in the case of phosphorescent emission that has a longer exciton life as compared with fluorescent emission, effects of the endothermic move of excitons relatively tend to appear. Relative to the thermal energy at room temperature, a larger energy difference ΔE^{T} is preferable. The energy difference ΔE^{T} is further preferably 0.1 eV or more, and particularly preferably 0.2 eV or more. On the other hand, in a fluorescent device, as the triplet barrier layer of the TTF device configuration disclosed in WO2010/134350A1, the material for an organic EL device according to the invention can be used.

The electron mobility of the material constituting the triplet barrier layer is desirably 10⁻⁶ cm²/Vs or more in a field intensity range of 0.04 to 0.5 MV/cm. As the method for measuring the electron mobility of an organic material, several methods that include the Time of Flight method are known. Here, the electron mobility means an electron mobility that is determined by the impedance spectroscopy.

The electron mobility of the electron-injecting layer is desirably 10⁻⁶ cm²Ns or more in a field intensity range of 0.04 to 0.5 MV/cm. The reason is that, by this electron mobility, injection of electrons from the cathode to the electron-transporting layer is promoted, and as a result, injection of electrons to adjacent barrier layer and emitting layer is promoted, enabling the device to be driven at a lower voltage.

The organic EL device of the invention can be used as an emitting device in a panel module used in various displays.

The organic EL device according to the invention can be used as a display element of a TV, a mobile phone and a PC; or an electronic apparatus such as lightings or the like.

### EXAMPLES

### [Synthesis of intermediates (A) to (J)]

### Synthesis Example 1 (Synthesis of intermediates (A) and (B))

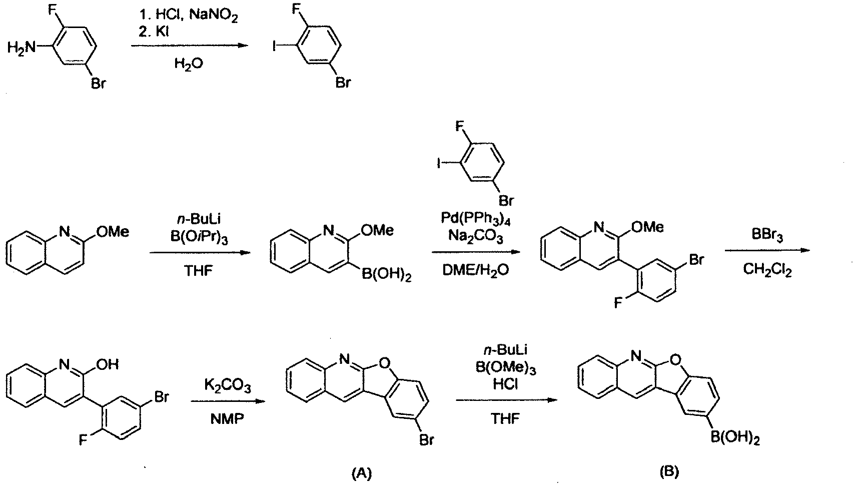

### (1) Synthesis of 5-bromo-2-fluoroiodobenzene

In an argon atmosphere, water (120 mL) was added to 5-bromo-2-fluoroaniline (45.6g, 240 mmol), followed by stirring. After adding concentrated hydrochloric acid (120 mL), the mixture was cooled to -20°C, and an aqueous solution of sodium sulfite (19.9g, 288 mmol) and water (80 mL) was added dropwise. The resultant was stirred at -20°C for 20 minutes, an aqueous solution of potassium iodide (59.8g, 360 mmol) and water (60 mL) was added, and stirred for 30 minutes. After extracting with hexane, an organic layer was washed with an aqueous solution of saturated sodium hydrogen carbonate and an aqueous solution of sodium sulfite, and dried with sodium sulfate. The solvent was removed under reduced pressure. Residues were purified with silica gel column chromatography, whereby 5-bromo-2-fluoroiodobenzene (54.6g, 181 mmol) (yield: 76%) was obtained.

### (2) Synthesis of 2-methoxyquinoline-3-boronic acid

2-methoxyquinoline (20.0g, 126 mmol) and triisopropoxyborane (59.1g, 314 mmol, tetrahydrofuran (dehydrated) (200 mL) were mixed, and cooled to -78°C. To the resultant, n-butyllithium (1.6M in hexane, 86 mL, 138mmol) was added, and stirred at -78°C for 4 hours, and then heated up to room temperature in 5 hours. After completion of the reaction, a saturated NH₄Cl solution (100 mL) was added, and a HCI solution (3M) was added until the pH became 5. Thereafter, solids obtained by concentrating the organic layer were washed by suspending them in water, and recovered by filtration, whereby 2-methoxyquinoline-3-boronic acid (19.7g, 97.0 mmol) was obtained (yield: 77%).

### (3) Synthesis of 2-methoxy-3-(2-fluoro-5-bromophenyl)quinoline

In an argon atmosphere, 2-methoxyquinoline-3-boronic acid (12.0g, 59.0 mmol), 5-bromo-2-fluoroiodobenzene (18.7g, 62.1 mmol), tetrakistriphenylphosphine palladium (1.37g, 1.18 mmol), 1,2-dimethoxyethane (100 mL) and an aqueous sodium carbonate solution (2M, 100 mL) were mixed. The resultant was heated under reflux for 6 hours. After cooling to room temperature, the reaction solution was extracted with toluene. After removing an aqueous layer, an organic layer was washed with saturated saline. The organic layer was dried with magnesium sulfate and concentrated. The residues were purified with silica gel column chromatography, whereby 2-methoxy-3-(2-fluoro-5-bromophenyl)quinoline (12.8g, 38.4 mmol) was obtained (yield: 65%).

### (4) Synthesis of 2-hydroxy-3-(2-fluoro-5-bromophenyl)quinoline

2-methoxy-3-(2-fluoro-5-bromophenyl)quinoline (12.0g, 36.1 mmol) and dichloromethane (dehydrated) (360 mL) were mixed, and cooled to -78°C. BBr₃(1M in dichloromethane, 54.2 mL, 54.2 mmol) was added, and then heated up to room temperature in 3 hours. After completion of the reaction, the solution was cooled to -78°C, carefully deactivated with methanol, and then deactivated with a sufficient amount of water. The solution was extracted with dichloromethane, dried with magnesium sulfiate, and then passed through a short silica gel chromatography. Thereafter, the solution was dried by concentration, whereby 2-hydroxy-3-(2-fluoro-5-bromophenyl)quinoline (5.05g, 15.9 mmol) (yield: 44%) was obtained.

### (5) Synthesis of intermediate (A)

2-hydroxy-3-(2-fluoro-5-bromophenyl)quinoline (7.30g, 22.9 mmol), N-methyl-2-pyrrolidinone (dehydrated) (70 mL) and K₂CO₃ (6.33g. 45.8 mmol) were mixed, and stirred at 120°C for 2 hours. After completion of the reaction, the solution was cooled to room temperature, diluted with toluene and washed with water. This solution was dried with magnesium sulfate, and then purified by silica gel column chromatography, whereby intermediate (A) (4.92g, 16. 5 mmol) (yield: 72%) was obtained.

### (6) Synthesis of intermediate (B)

Intermediate (A) (2.65g, 8.90 mmol) and tetrahydrofuran (dehydrated) (100 mL) were mixed, and cooled to -78°C. Thereafter, n-butyl (n-Bu) Li (1.60M in hexane, 5.85 mL, 9.35 mmol) was added, and heated up to 0°C in 2 hours. Then, the solution was again cooled to -78°C. Trimethoxyboran (2.31g, 22.3 mmol) was added, and stirred at -78°C for 10 minutes. The resultant was heated up to room temperature in 5 hours. After completion of the reaction, a HCI solution (1M, 25 mL) was added, stirred at room temperature for 1 hour, and then extracted with ethyl acetate. This solution was dried with magnesium sulfate, concentrated, washed by suspending it in hexane and recovered by filtration, whereby intermediate (B) (960 mg, 3.65 mmol) (yield: 41%) was obtained.

### Synthtesis Example 2 (synthesis of intermediates (C) and (D))

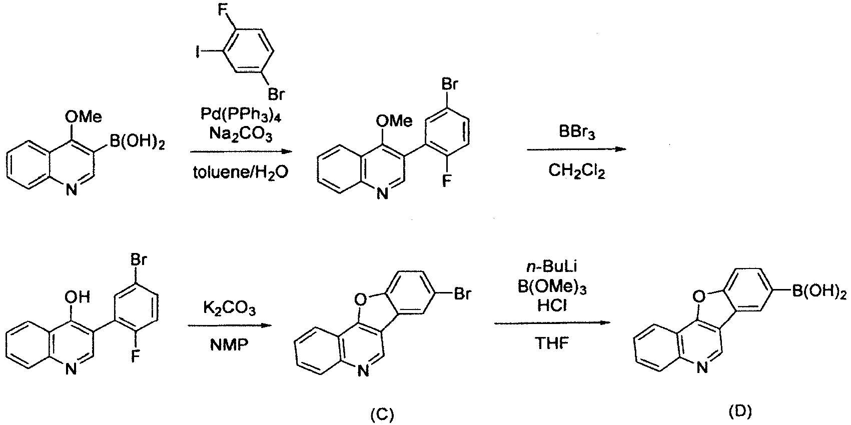

Intermediate (C) and intermediate (D) were synthesized according to the above-mentioned scheme in the same manner as in the synthesis of intermediate (B), except that 4-methoxyquinoline-3-boronic acid was used instead of 2-methoxyquinoline-3-boronic acid.

### Synthesis Example 3 (synthesis of intermediates (E) and (F))

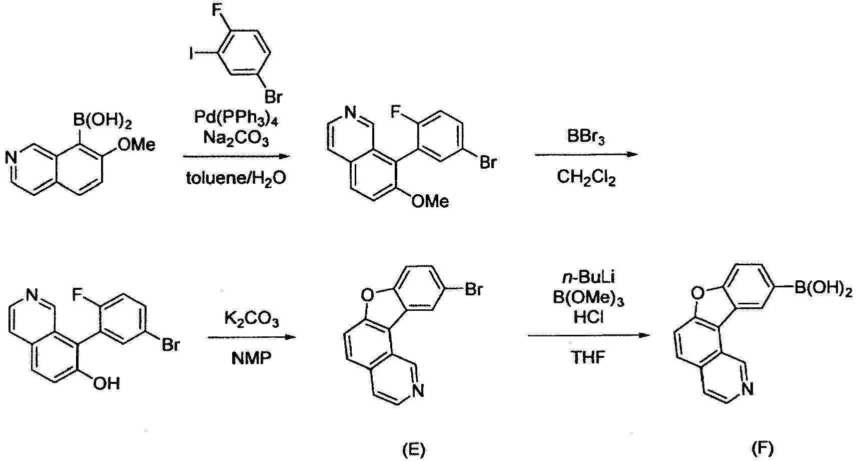

Intermediate (E) and intermediate (F) were synthesized according to the above-mentioned scheme in the same manner as in the synthesis of intermediate (B), except that 7-methoxyisoquinoline-8-boronic acid was used instead of 2-methoxyquinoline-3-boronic acid.

### Synthesis Example 4 (synthesis of intermediates (G) and (H))

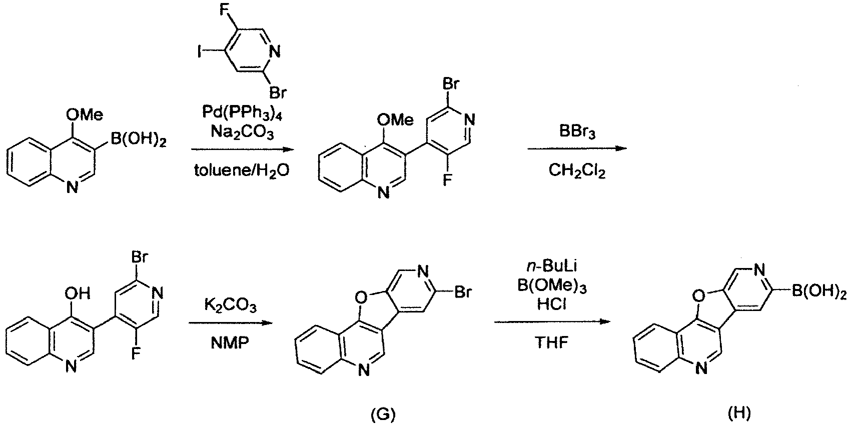

Intermediate (G) and intermediate (H) were synthesized according to the above-mentioned scheme in the same manner as in the synthesis of intermediate (D), except that 2-bromo-5-fluoro-4-iodopyridine was used instead of 5-bromo-2-fluoroiodobenzene.

### Synthesis Example 5 (synthesis of intermediates (I) and (J))

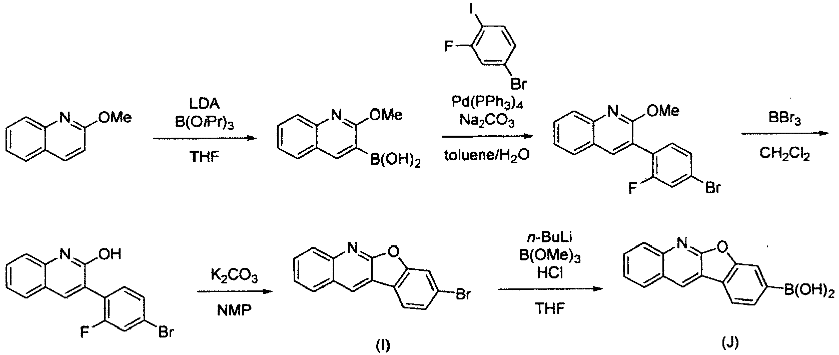

Intermediate (I) and intermediate (J) were synthesized according to the above-mentioned scheme in the same manner as in the synthesis of intermediate (B), except that 4-bromo-2-fluoroiodobenzene was used instead of 5-bromo-2-fluoroiodobenzene.

### [Synthesis of azanaphthobenzofuran derivative]

### Synthesis Example 6 (synthesis of compound 1)

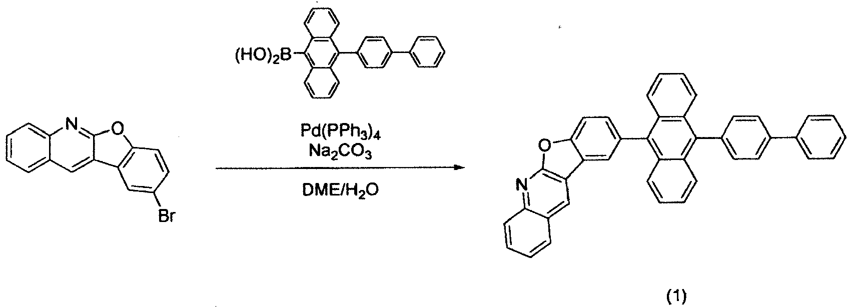

In an argon atmosphere, intermediate (A) (2.00g, 6.71 mmol), 10-(4-biphenyl)anthracene-9-boronic acid (2.51g, 6.71 mmol), tetrakis(triphenylphosphine)palladium (388 mg, 0.336 mmol), 1,2-dimethoxyethane (20 mL) and an aqueous sodium carbonate solution (2M, 20 mL) were mixed, and stirred while heating under reflux for 8 hours. After cooling to room temperature, precipitated solids were collected by filtration. The thus obtained solids were washed with water and methanol, and recrystallized from toluene, whereby compound 1 (2.02g, 3.69 mmol) (yield: 55%) was obtained. As a result of mass spectrometry, this compound was an intended product, and had an m/e value of 547 relative to a molecular weight of 547.66.

### Synthesis Example 7 (synthesis of compound 2)

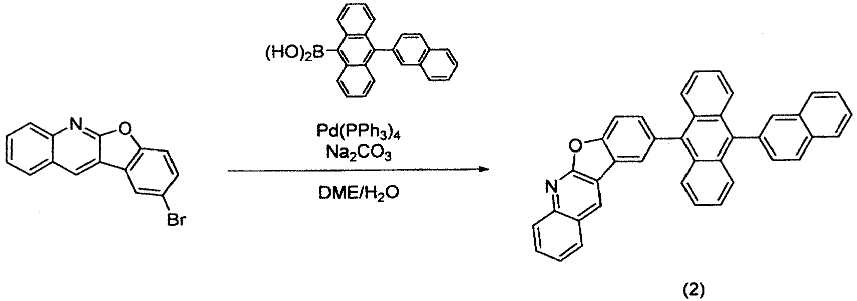

Synthesis was conducted in the same manner as in the synthesis of compound 1, except that 10-(2-naphthyl)anthrathene-9-boronic acid that had been synthesized by a known method was used instead of 10-(4-biphenyl)anthracene-9-boronic acid, whereby compound 2 was obtained. As a result of mass spectrometry, this compound was an intended product, and had an m/e value of 521 relative to a molecular weight of 521.62.

### Synthesis Example 8 (synthesis of compound 3) (Reference)

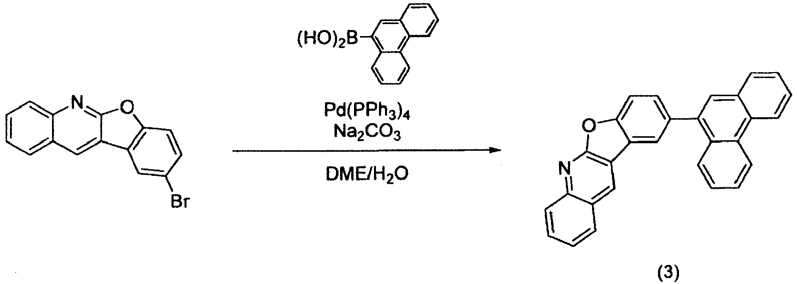

Synthesis was conducted in the same manner as in the synthesis of compound 1, except that 9-phenanthreneboronic acid that had been synthesized by a known method was used instead of 10-(4-biphenyl)anthracene-9-boronic acid, whereby compound 3 was obtained. As a result of mass spectrometry, this compound was an intended product, and had an m/e value of 395 relative to a molecular weight of 395.46.

### Synthesis Example 9 (synthesis of compound 4) (Reference)

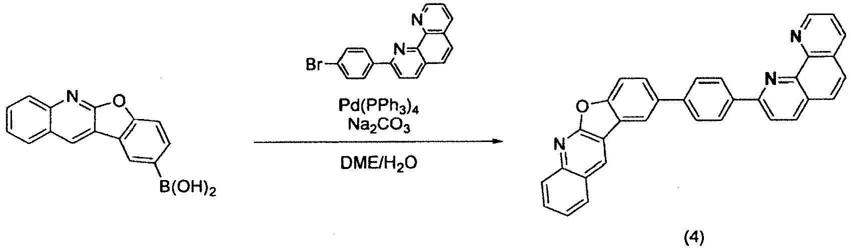

Synthesis was conducted in the same manner as in the synthesis of compound 1, except that 2-(4-bromophenyl)-1,10-phenanthroline that had been synthesized by a known method was used instead of intermediate A and intermediate B was used instead of 10-(4-biphenyl)anthracene-9-boronic acid, whereby compound 4 was obtained. As a result of mass spectrometry, this compound was an intended product, and had an m/e value of 473 relative to a molecular weight of 473.54.

### Synthesis Example 10 (synthesis of compound 5) (Reference)

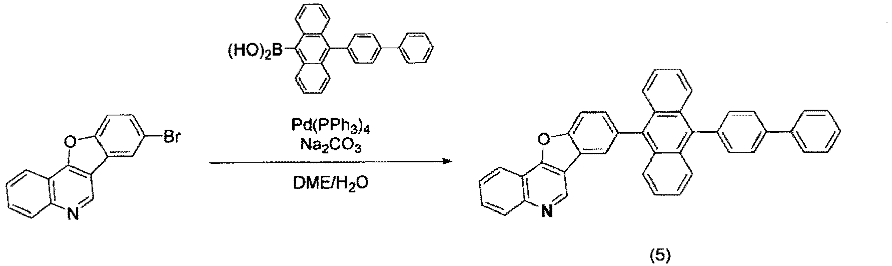

Synthesis was conducted in the same manner as in the synthesis of compound 1, except that intermediate C was used instead of intermediate A, whereby compound 5 was obtained. As a result of mass spectrometry, this compound was an intended product, and had an m/e value of 547 relative to a molecular weight of 547.66.

### Synthesis Example 11 (synthesis of compound 6) (Reference)

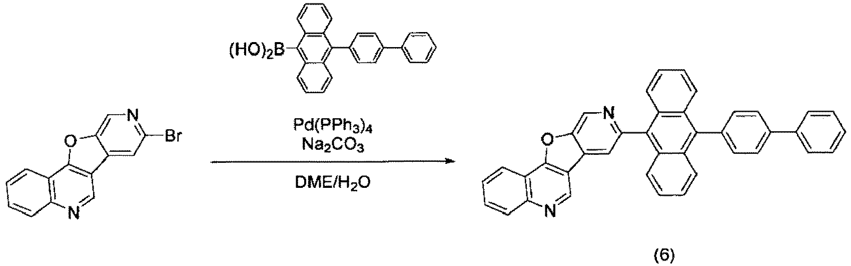

Synthesis was conducted in the same manner as in the synthesis of compound 1, except that intermediate G was used instead of intermediate A, whereby compound 6 was obtained. As a result of mass spectrometry, this compound was an intended product, and had an m/e value of 548 relative to a molecular weight of 548.64.

### Synthesis Example 12 (synthesis of compound 7) (Reference)

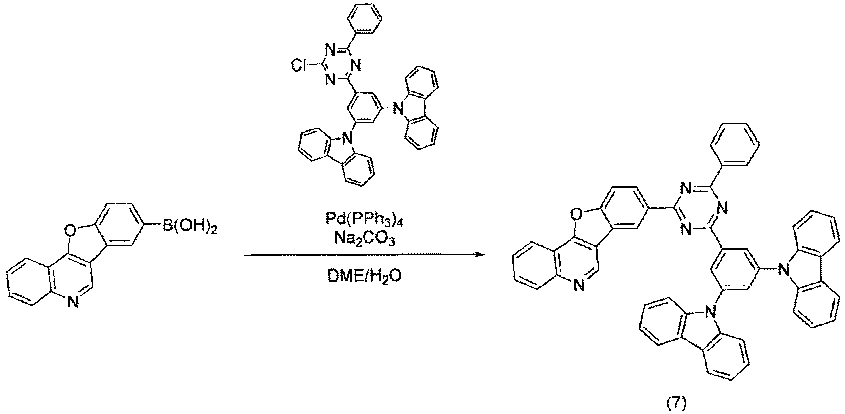

Synthesis was conducted in the same manner as in the synthesis of compound 1, except that 2-(3,5-biscarbazolylphenyl)-4-chloro-6-phenyl-[1,3,5]triazine that had been syntheized by a known method was used instead of intermediate A and intermediate D was used instead of 10-(4-biphenyl)anthracene-9-boronic acid, whereby compound 7 was obtained. As a result of mass spectrometry, this compound was an intended product, and had an m/e value of 780 relative to a molecular weight of 780.89.

### Synthesis Example 13 (synthesis of compound 8) (Reference)

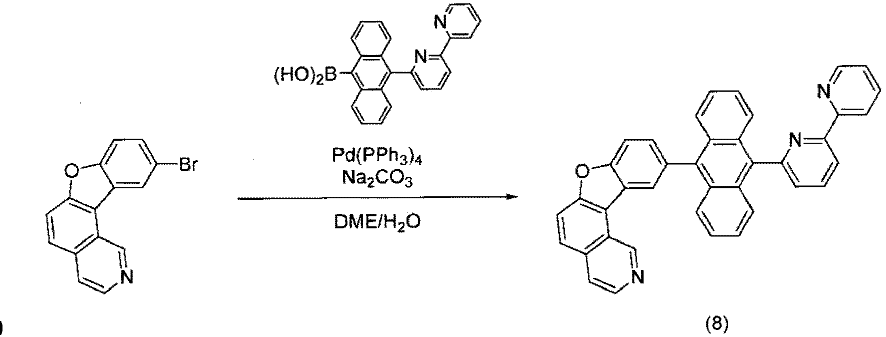

Synthesis was conducted in the same manner as in the synthesis of compound 1, except that inetermediate E was used instead of intermediate A and 10-(2,2'-bipyridin-6-yl)anthranene-9-boronic acid that had been synthesized by a known method was used instead of 10-(4-biphenyl)anthracene-9-boronic acid, whereby compound 8 was obtained. As a result of mass spectrometry, this compound was an intended product, and had an m/e value of 780 relative to a molecular weight of 780.89.

### Synthesis Example 14 (synthesis of compound 9) (Reference)

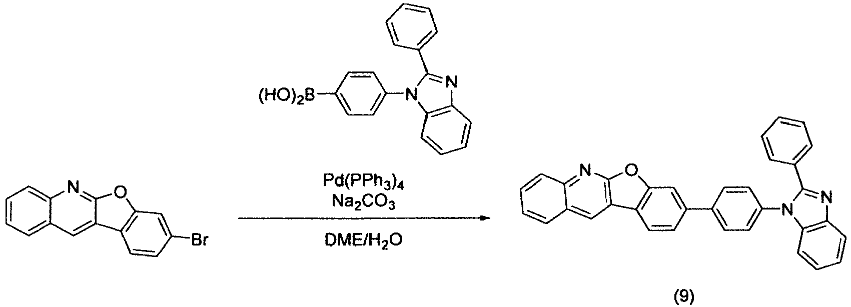

Synthesis was conducted in the same manner as in the synthesis of compound 1, except that inetermediate I was used instead of intermediate A and (4-(2-phenyl-1-H-benzo[d]imidazol-1-yl)phenyl)boronic acid that had been synthesized by a known method was used instead of 10-(4-biphenyl)anthracene-9-boronic acid, whereby compound 9 was obtained. As a result of mass spectrometry, this compound was an intended product, and had an m/e value of 487 relative to a molecular weight of 487.56.

### [Organic EL Device]

### Example 1 (Reference)

A glass substrate of 25 mm by 75 mm by 1.1 mm thick with an ITO transparent electrode (anode) (manufactured by GEOMATEC Co., Ltd.) was subjected to ultrasonic cleaning with isopropyl alcohol for 5 minutes, and then subjected to UV-ozone cleaning for 30 minutes. The cleaned glass substrate with a transparent electrode was mounted in a substrate holder of a vacuum vapor deposition apparatus. First, compound A-1 was deposited on the surface where transparent electrode lines were formed so as to cover the transparent electrode, thereby to form a 60 nm-thick compound A-1 film. Subsequent to the formation of the A-1 film, a 20 nm-thick A-2 film was formed on this A-1 film.

Further, on this A-2 film, the following host H-1 and dopant D-1 were formed into a 40 nm-thick film in a film thickness ratio of 40:2, whereby an emitting layer was formed. On the emitting layer, as an electron-transporting layer, compound 3 synthezied in Synthesis Example 8 was formed into a 20 nm-thick film by deposition. Thereafter, LiF was formed into a 1 nm-thick film, and metal Al was deposited on this LiF film in a thickness of 150 nm to form a metal cathode, whereby an organic EL device was formed.

The organic EL device as fabricated above was allowed to emit light by passing through DC current of 10 mA/cm², and driving voltage and luminous efficiency were measured. The half life was measured by conducting a continuous DC current test at an initial luminance of 1000 cd/m². The results of the driving voltage and the half life are shown in Table 1.

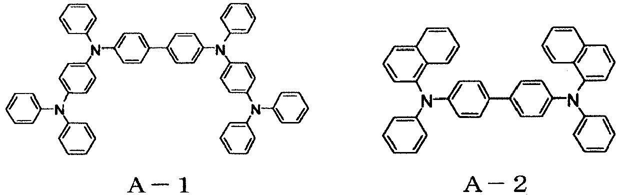

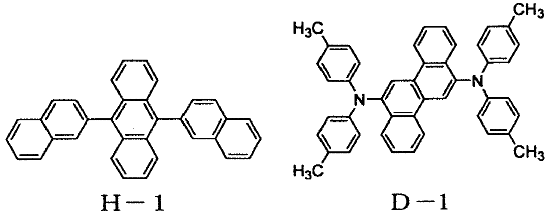

### Comparative Example 1

An organic EL device was prepared and evaluated in the same manner as in Example 1, except that compound (A) with the following structure was used instead of compound 3. The results are shown in Table 1.

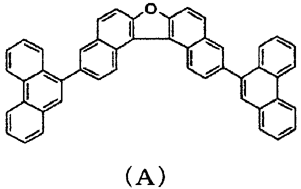

**[Table 1**

| | Electron-transporting layer | Voltage (V) (at the time of 10 mNcm2) | Lifetime (h) | Emission color |
|---|---|---|---|---|
| Example 1 | Compound 3 | 4.8 | 2000 | Blue |
| Comparative Example 1 | Compound A | 8.0 | 1000 | Blue |

From the results mentioned above, it can be confirmed that, by using the compound 3, a lowering in driving voltage of an organic EL device can be realized. As compared with the compound (A) used in Comparative Example 1, an organic EL device obtained by using an azidated naphthobenzofuran derivative can be driven at a low voltage.

### Example 2 (according to the invention)

Formation of films was conducted in the same manner as in Example 1, until the emitting layer was formed. On the emitting layer, as an electron-transporting layer, compound 1 synthesized in the above-mentioned Synthesis Example 6 and lithium 8-hydroxyquinolate (Liq) were co-deposited in a film thickness ratio of 1:1, whereby a 35 nm-thick film was formed. On this electron-transporting layer, metal Al was deposited in a thickness of 80 nm to form a metal cathode, whereby an organic EL device was fabricated. Evaluation was conducted in the same manner as in Example 1. The results of the driving voltage and the luminous efficiency are shown in Table 2.

### Comparative Example 2

An organic EL device was fabricated and evaluated in the same manner as in Example 2, except that compound (B) having the following structure was used instead of compound 1. The results are shown in Table 2.

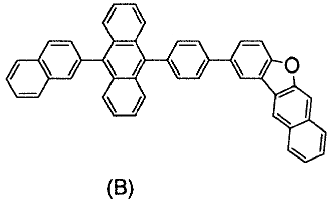

**[Table 2]**

| | Electron-transporting layer | Voltage (V) (at the time of 10 mA/cm²) | Efficiency (%) | Emission color |
|---|---|---|---|---|
| Example 2 | Compound 1 | 4.8 | 8.7 | Blue |
| Comparative Example 2 | Compound B | 6.1 | 7.9 | Blue |

From the above, by using the compound of the invention in which the naphthobenzofuran skeleton that has been azidated, a lowering in voltage and an increase in efficiency in an organic EL device can be realized.

Although only some exemplary embodiments and/or examples of this invention have been described in detail above, those skilled in the art will readily appreciate that many modifications are possible in the exemplary embodiments and/or examples within the scope of the appended claims.

## Claims

1. A material for an organic electroluminescence device, comprising a compound represented by the following formula (2-1'): 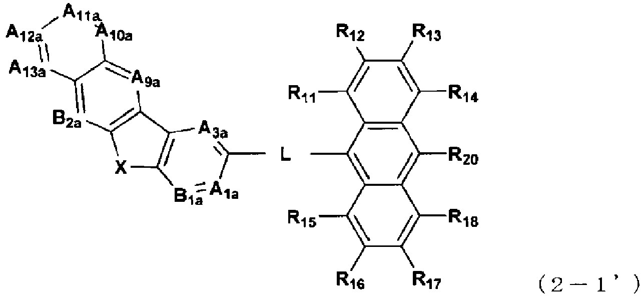 wherein in the formula,
X is a sulfur atom S or an oxygen atom O;
A₁ₐ, A₃ₐ, A₉ₐ-A₁₃ₐ are independently CH orCR₁, wherein R₁ is a substituent, independently selected from a group consisting of
a substituted or unsubstituted alkyl group including 1 to 50 carbon atoms;
a substituted or unsubstituted cycloalkyl group including 3 to 50 ring carbon atoms;
a substituted or unsubstituted aryl group including 6 to 50 ring carbon atoms selected from phenyl, naphthyl, naphthylphenyl, biphenylyl, terphenylyl, acenaphthylenyl, anthryl, benzoanthryl, aseanthryl, phenanthryl, benzophenanthryl, phenalenyl, fluorenyl, 9,9'-spirobifluorenyl, benzofluorenyl, dibenzofluorenyl, picenyl, pentaphenyl, pentacenyl, pyrenyl, chrysenyl, benzochrysenyl, s-indacenyl, as-indacenyl, fluoranthenyl, benzofluoranthenyl, tetracenyl, triphenylenyl, benzotriphenylenyl, perylenyl, coronyl, and dibenzoanthryl;
a substituted or unsubstituted aralkyl group including 7 to 51 carbon atoms;
an amino group;
a mono-substituted or di-substituted amino group having a substituent selected from a substituted or unsubstituted alkyl group including 1 to 50 carbon atoms or a substituted or unsubstituted aryl group including 6 to 50 ring carbon atoms;
a mono-substituted, di-substituted or tri-substituted silyl group having a substituent selected from a substituted or unsubstituted alkoxyl group including 1 to 50 carbon atoms, a substituted or unsubstituted aryloxy group including 6 to 50 ring carbon atoms, a substituted or unsubstituted alkyl group including 1 to 50 carbon atoms and a substituted or unsubstituted aryl group including 6 to 50 ring carbon atoms; a substituted or unsubstituted heteroaryl group including 5 to 50 ring atoms, a substituted or unsubstituted haloalkyl group including 1 to 50 carbon atoms;
a halogen atom;
a cyano group;
a nitro group;
a sulfonyl group having a substituent selected from a substituted or unsubstituted alkyl group including 1 to 50 carbon atoms and a substituted or unsubstituted aryl group including 6 to 50 ring carbon atoms;
a di-substituted phosphoryl group having a substituent selected from a substituted or unsubstituted alkyl group including 1 to 50 carbon atoms, a substituted or unsubstituted heteroaryl group including 5 to 50 ring atoms and a substituted or unsubstituted aryl group including 6 to 50 ring carbon atoms;
an alkylsufonyloxy group;
an arylsulfonyloxy group;
an alkylcarbonyloxy group;
an arylcarbonyloxy group;
a boron-containing group;
a zinc-containing group;
a tin-containing group;
a silicon-containing group;
a magnesium-containing group;
a lithium-containing group;
a hydroxy group;
an alkyl-substituted or aryl-substituted carbonyl group;
a carboxy group;
a vinyl group;
a (meth)acryloyl group;
an epoxy group; and
an oxetanyl group;
B₁ₐ is CH, CR₂ or a nitrogen atom N, and R₂ is a substituted or unsubstituted alkyl group including 1 to 50 carbon atoms, a substituted or unsubstituted haloalkyl group including 1 to 50 carbon atoms, a cyano group or a halogen atom;
B₂ₐ is a nitrogen atom N;
R₁₁ to R₁₈ are independently a hydrogen atom or a substituent, which is the same as that of R₁; R₂₀ is a substituted or unsubstituted aryl group including 6 to 50 ring carbon atoms or a substituted or unsubstituted heteroaryl group including 5 to 50 ring atoms;
L is a single bond or a substituted or unsubstituted aromatic ring.

2. An organic electroluminescence device which comprises a cathode, an anode, and at least one organic thin film layer including an emitting layer disposed between the cathode and the anode, wherein at least one of said organic thin film layers comprises the material for an organic electroluminescence device according to claim 1.

3. The organic electroluminescence device according to claim 2, wherein the emitting layer comprises the material for an organic electroluminescence device.

4. The organic electroluminescence device according to claim 2 or 3, which further comprises an anode-side organic thin film layer disposed between the anode and the emitting layer, and the anode-side organic thin film layer contains the material for an organic electroluminescence device.

5. The organic electroluminescence device according any one of claims 2 to 4, which further comprises a cathode-side organic thin film layer disposed between the cathode and the emitting layer, and the cathode-side organic thin film layer contains the material for an organic electroluminescence device.

6. The organic electroluminescence device according to any one of claims 2 to 5, wherein the emitting layer contains a fluorescent emitting material.

7. The organic electroluminescence device according to any one of claims 2 to 5, wherein the emitting layer contains a phosphorescent emitting material.

8. The organic electroluminescence device according to claim 7, wherein the phosphorescent emitting material is an ortho-metalated complex of a metal atom selected from iridium (Ir), osmium (Os) and platinum (Pt).

9. An electronic apparatus comprising the organic electroluminescence device according to any one of claims 2 to 8.

## Patentansprüche

1. Material für eine organische Elektrolumineszenzvorrichtung, enthaltend eine Verbindung mit der folgenden Formel (2-1'): 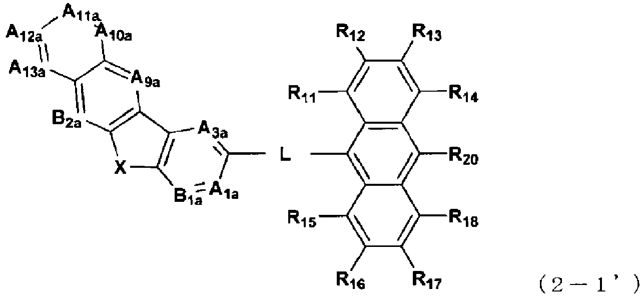 worin in der Formel
X ein Schwefelatom S oder ein Sauerstoffatom O ist,
A₁ₐ , A₃ₐ , A₉ₐ-A₁₃ₐ jeweils unabhängig CH oder CR₁ sind, worin R₁ ein Substituent ist, unabhängig ausgewählt aus der Gruppe, bestehend aus:
einer substituierten oder unsubstituierten Alkylgruppe, enthaltend 1 bis 50 Kohlenstoffatome,
einer substituierten oder unsubstituierten Cycloalkylgruppe, enthaltend 3 bis 50 Ringkohlenstoffatome,
einer substituierten oder unsubstituierten Arylgruppe, enthaltend 6 bis 50 Ringkohlenstoffatome, ausgewählt aus Phenyl, Naphthyl, Naphthylphenyl, Biphenylyl, Terphenylyl, Acenaphthylenyl, Anthryl, Benzoanthryl, Aseanthryl, Phenanthryl, Benzophenanthryl, Phenalenyl, Fluorenyl, 9,9'-Spirobifluorenyl, Benzofluorenyl, Dibenzofluorenyl, Picenyl, Pentaphenyl, Pentacenyl, Pyrenyl, Chrysenyl, Benzochrysenyl, s-Indacenyl, as-Indacenyl, Fluoranthenyl, Benzofluoranthenyl, Tetracenyl, Triphenylenyl, Benzotriphenylenyl, Perylenyl, Coronyl und Dibenzoanthryl,
einer substituierten oder unsubstituierten Aralkylgruppe, enthaltend 7 bis 51 Kohlenstoffatome,
einer Aminogruppe,
einer mono-substituierten oder di-substituierten Aminogruppe mit einem Substituenten, ausgewählt aus einer substituierten oder unsubstituierten Alkylgruppe mit 1 bis 50 Kohlenstoffatomen oder substituierten oder unsubstituierten Arylgruppe mit 6 bis 50 Ringkohlenstoffatomen,
einer mono-substituierten, di-substituierten oder trisubstituierten Silylgruppe mit einem Substituenten,
ausgewählt aus einer substituierten oder unsubstituierten Alkoxylgruppe mit 1 bis 50 Kohlenstoffatomen, substituierten oder unsubstituierten Aryloxygruppe mit 6 bis 50 Ringkohlenstoffatomen,
substituierten oder unsubstituierten Alkylgruppe mit 1 bis 50 Kohlenstoffatomen oder substituierten oder
unsubstituierten Arylgruppe mit 6 bis 50 Ringkohlenstoffatomen, substituierten oder
unsubstituierten Heteroarylgruppe mit 5 bis 50 Ringatomen, substituierten oder unsubstituierten Haloalkylgruppe mit 1 bis 50 Kohlenstoffatomen,
Halogenatom,
Cyanogruppe,
Nitrogruppe,
Sulfonylgruppe mit einem Substituenten, ausgewählt aus einer substituierten oder unsubstituierten Alkylgruppe mit 1 bis 50 Kohlenstoffatomen und einer substituierten oder unsubstituierten Arylgruppe mit 6 bis 50 Ringkohlenstoffatomen,
einer di-substituierten Phosphorylgruppe mit einem Substituenten, ausgewählt aus einer substituierten oder
unsubstituierten Alkylgruppe mit 1 bis 50 Kohlenstoffatomen, substituierten oder unsubstituierten Heteroarylgruppe mit 5 bis 50 Ringatomen und
substituierten oder unsubstituierten Arylgruppe mit 6 bis 50 Ringkohlenstoffatomen,
Alkylsufonyloxygruppe,
Arylsulfonyloxygruppe,
Alkylcarbonyloxygruppe,
Arylcarbonyloxygruppe,
Bor-haltigen Gruppe,
Zink-haltigen Gruppe,
Zinn-haltigen Gruppe,
Silicium-haltigen Gruppe,
Magnesium-haltigen Gruppe,
Lithium-haltigen Gruppe,
Hydroxygruppe,
Alkyl-substitutierten oder Aryl-substituierten Carbonylgruppe,
Carboxygruppe,
Vinylgruppe,
(Meth)acryloylgruppe,
Epoxygruppe und
Oxetanylgruppe,
B₁ₐ CH, CR₂ oder ein Stickstoffatom N ist und R₂ eine substituierte oder unsubstituierte Alkylgruppe mit 1 bis 50 Kohlenstoffatomen, substituierte oder unsubstituierte Haloalkylgruppe mit 1 bis 50 Kohlenstoffatomen,
Cyanogruppe oder Halogenatom ist,
B₂ₐ Stickstoffatom N ist,
R₁₁ bis R₁₈ unabhängig ein Wasserstoffatom oder ein Substituent sind, der gleich ist wie bei R₁,
R₂₀ eine substituierte oder unsubstituierte Arylgruppe mi 6 bis 50 Ringkohlenstoffatomen oder substituierte oder unsubstituierte Heteroarylgruppe mit 5 bis 50 Ringatomen ist,
L eine Einfachbindung oder ein substituierter oder
unsubstituierter aromatischer Ring ist.

2. Organische Elektrolumineszenzvorrichtung, enthaltend eine Kathode, eine Anode und zumindest eine organische Dünnfilmschicht, enthaltend eine emittierende Schicht, die zwischen der Kathode und der Anode angeordnet ist, worin zumindest eine der organischen Dünnfilmschichten das Material für eine organische Elektrolumineszenzvorrichtung gemäß Anspruch 1 enthält.

3. Organische Elektrolumineszenzvorrichtung gemäß Anspruch 2, worin die emittierende Schicht das Material für eine organische Elektrolumineszenzvorrichtung enthält.

4. Organische Elektrolumineszenzvorrichtung gemäß Anspruch 2 oder 3, die weiterhin eine anodenseitige organische Dünnfilmschicht enthält, die zwischen der Anode und der emittierenden Schicht angeordnet ist, und die anodenseitige organische Dünnfilmschicht das Material für eine organische Elektrolumineszenzvorrichtung enthält.

5. Organische Elektrolumineszenzvorrichtung gemäß einem der Ansprüche 2 bis 4, die weiterhin eine kathodenseitige organische Dünnfilmschicht enthält, die zwischen der Kathode und der emittierenden Schicht angeordnet ist und worin die kathodenseitige organische Dünnfilmschicht das Material für eine organische Elektrolumineszenzvorrichtung enthält.

6. Organische Elektrolumineszenzvorrichtung gemäß einem der Ansprüche 2 bis 5, worin die emittierende Schicht ein fluoreszenzemittierendes Material enthält.

7. Organische Elektrolumineszenzvorrichtung gemäß einem der Ansprüche 2 bis 5, worin die emittierende Schicht ein phosphoreszenemittierendes Material enthält.

8. Organische Elektrolumineszenzvorrichtung gemäß Anspruch 7, worin das phosphoreszenzemittierende Material ein ortho-metallisierter Komplex aus einem Metallatom ist, ausgewählt aus Iridium (Ir), Osmium (OS) und Platin (Pt) .

9. Elektronische Anlage, enthaltend die organische Elektrolumineszenzvorrichtung gemäß einem der Ansprüche 2 bis 8.

## Revendications

1. Matériau pour un dispositif électroluminescent organique comprenant un composé représenté par la formule (2-1') suivante : 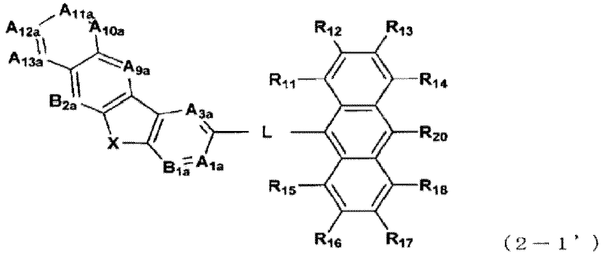 dans lequel dans la formule,
X est un atome de soufre S ou un atome d'oxygène O ;
A₁ₐ, A₃ₐ, A₉ₐ-A₁₃ₐ sont indépendamment CH ou CR₁, dans lequel R₁ est un substituant, indépendamment sélectionné dans un groupe constitué par
un groupe alkyle substitué ou non substitué incluant 1 à 50 atomes de carbone ;
un groupe cycloalkyle substitué ou non substitué incluant 3 à 50 atomes de carbone cycliques ;
un groupe aryle substitué ou non substitué incluant 6 à 50 atomes de carbone cycliques sélectionnés parmi le phényle, le naphtyle, le naphtylphényle, le biphénylyle, le terphénylyle, l'acénaphtylényle, l'anthryle, le benzoanthryle, l'aséanthryle, le phénanthryle, le benzophénanthryle, le phénalényle, le fluorényle, le 9,9'-spirobifluorényle, le benzofluorényle, le dibenzofluorényle, le picényle, le pentaphényle, le pentacényle, le pyrényle, le chrysényle, le benzochrysényle, le s-indacényle, l'as-indacényle, le fluoranthényle, le benzofluoranthényle, le tétracényle, le triphénylényle, le benzotriphénylényle, le pérylényle, le coronyle et le dibenzoanthryle ;
un groupe aralkyle substitué ou non substitué incluant 7 à 51 atomes de carbone ;
un groupe amino ;
un groupe amino monosubstitué ou disubstitué présentant un substituant sélectionné parmi un groupe alkyle substitué ou non substitué incluant 1 à 50 atomes de carbone ou un groupe aryle substitué ou non substitué incluant 6 à 50 atomes de carbone cycliques ;
un groupe silyle monosubstitué, disubstitué ou trisubstitué présentant un substituant sélectionné parmi un groupe alcoxyle substitué ou non substitué incluant 1 à 50 atomes de carbone, un groupe aryloxy substitué ou non substitué incluant 6 à 50 atomes de carbone cycliques, un groupe alkyle substitué ou non substitué incluant 1 à 50 atomes de carbone et un groupe aryle substitué ou non substitué incluant 6 à 50 atomes de carbone cycliques ;
un groupe hétéroaryle substitué ou non substitué incluant 5 à 50 atomes cycliques, un groupe haloalkyle substitué ou non substitué incluant 1 à 50 atomes de carbone ;
un atome d'halogène ;
un groupe cyano ;
un groupe nitro ;
un groupe sulfonyle présentant un substituant sélectionné parmi un groupe alkyle substitué ou non substitué incluant 1 à 50 atomes de carbone et un groupe aryle substitué ou non substitué incluant 6 à 50 atomes de carbone cycliques ;
un groupe phosphoryle disubstitué présentant un substituant sélectionné parmi un groupe alkyle substitué ou non substitué incluant 1 à 50 atomes de carbone, un groupe hétéroaryle substitué ou non substitué incluant 5 à 50 atomes cycliques et un groupe aryle substitué ou non substitué incluant 6 à 50 atomes de carbone cycliques ;
un groupe alkylsulfonyloxy ;
un groupe arylsulfonyloxy ;
un groupe alkylcarbonyloxy ;
un groupe arylcarbonyloxy ;
un groupe contenant du bore ;
un groupe contenant du zinc ;
un groupe contenant de l'étain ;
un groupe contenant du silicium ;
un groupe contenant du magnésium ;
un groupe contenant du lithium ;
un groupe hydroxy ;
un groupe carbonyle substitué par alkyle ou substitué par aryle ;
n groupe carboxy ;
un groupe vinyle ;
un groupe (méth)acryloyle ;
un groupe époxy ; et
un groupe oxétanyle ;
B₁ₐ est CH, CR₂ ou un atome d'azote N, et R₂ est un groupe alkyle substitué ou non substitué incluant 1 à 50 atomes de carbone, un groupe haloalkyle substitué ou non substitué incluant 1 à 50 atomes de carbone, un groupe cyano ou un atome d'halogène ;
B₂ₐ est un atome d'azote N ;
R₁₁ à R₁₈ sont indépendamment un atome d'hydrogène ou un substituant, qui est le même que celui de R₁ ;
R₂₀ est un groupe aryle substitué ou non substitué incluant 6 à 50 atomes de carbone cycliques ou un groupe hétéroaryle substitué ou non substitué incluant 5 à 50 atomes cycliques ;
L est une liaison simple ou un cycle aromatique substitué ou non substitué.

2. Dispositif électroluminescent organique qui comprend une cathode, une anode, et au moins une couche de film mince organique incluant une couche émissive disposée entre la cathode et l'anode, dans lequel au moins l'une desdites couches de film mince organiques comprend le matériau pour un dispositif électroluminescent organique selon la revendication 1.

3. Dispositif électroluminescent organique selon la revendication 2, dans lequel la couche émissive comprend le matériau pour un dispositif électroluminescent organique.

4. Dispositif électroluminescent organique selon la revendication 2 ou 3, qui comprend en outre une couche de film mince organique côté anode disposée entre l'anode et la couche émissive, et la couche de film mince organique côté anode contient le matériau pour un dispositif électroluminescent organique.

5. Dispositif électroluminescent organique selon l'une quelconque des revendications 2 à 4, qui comprend en outre une couche de film mince organique côté cathode disposée entre la cathode et la couche émissive, et la couche de film mince organique côté cathode contient le matériau pour un dispositif électroluminescent organique.

6. Dispositif électroluminescent organique selon l'une quelconque des revendications 2 à 5, dans lequel la couche émissive contient un matériau émissif fluorescent.

7. Dispositif électroluminescent organique selon l'une quelconque des revendications 2 à 5, dans lequel la couche émissive contient un matériau émissif phosphorescent.

8. Dispositif électroluminescent organique selon la revendication 7, dans lequel le matériau émissif phosphorescent est un complexe ortho-métalaté d'un atome métallique sélectionné parmi l'indium (Ir), l'osmium (Os) et le platine (Pt)

9. Appareil électronique comprenant le dispositif électroluminescent organique selon l'une quelconque des revendications 2 à 8.
